# FASCICULE DE BREVET EUROPEEN

(11) **EP 2 344 486 B1**
(45) Date de publication et mention de la délivrance du brevet: **27.03.2013**
(21) Numéro de dépôt: 09737098.5
(22) Date de dépôt: 21.07.2009
(51) Int. Cl.: C07D 417/14, A61K 31/4535, A61K 31/4709, A61P 29/00, A61P 1/08, A61K 31/427, A61P 25/00, A61P 19/10, A61P 33/00, A61P 35/00, C07D 213/74, C07D 211/26, C07D 277/24, A61K 31/4545, A61P 9/00, A61P 37/00, A61P 1/00

(54) **DERIVES DE CARBAMATES D'ALKYLTHIAZOLES, LEUR PREPARATION ET LEUR UTILISATION COMME INHBITEURS DE L'ENZYME FAAH**
ALKYLTHIAZOLCARBAMAT-VERBINDUNGEN, DEREN HERSTELLUNGSVERFAHREN UND VERWENDUNG ALS FAAH HEMMER
ALKYLTHIAZOL CARBAMATE DERIVATIVES, PREPARATION THEREOF AND THEIR USE AS FAAH INHIBITORS

(30) Priorité: 23.07.2008 FR 0804179
(43) Date de publication de la demande: 20.07.2011
(73) Titulaire: SANOFI, 75008 Paris (FR)
(72) Inventeur: ABOUABDELLAH, Ahmed, F-75013 Paris (FR); GÖRLITZER, Jochen, 65926 Frankfurt (DE); HAMLEY, Peter, 65926 Fankfurt (DE); RAVET, Antoine, F-75013 Paris (FR)
(74) Mandataire: Le Coupanec, Pascale A.M.P.
(86) Numéro de dépôt international: PCT/FR2009/051457
(87) Numéro de publication internationale: WO 2010/010288

(56) Documents cités:
- EP-A- 1 780 210
- FR-A- 2 854 633

## Description

L'invention a pour objet des dérivés de carbamates d'alkylthiazoles, leur préparation et leur application en thérapeutique.

Il existe toujours une nécessité de trouver et de développer des produits inhibiteurs de l'enzyme FAAH. Les composés de l'invention répondent à ce but.

Les composés de l'invention répondent à la formule générale (I) : 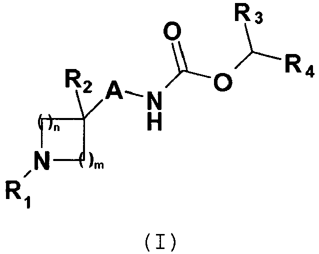 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
n représente un nombre entier égal à 1, 2 ou 3 et m représente un nombre entier égal à 1 ou 2;
A représente une liaison covalente ou un groupe C₁₋₈-alkylène ;
R₁ représente un groupe R₅ éventuellement substitué par un ou plusieurs groupes R₆ et/ou R₇ ;
   R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
   R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
   R₇ représente un groupe choisi parmi un furanyle, pyrrolyle, thiènyle, oxazolyle, isoxazolyle, thiazolyle, isothiazolyle, imidazolyle, pyrazolyle,oxadiazole, thiadiazole, phényle, pyridinyle, pyridazinyle, pyrimidinyle, pyrazinyle, triazine, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un thiazole éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉, -O- (C₁₋₃-alkylène) -O-, phényle, phényloxy, benzyloxy, pyridinyle, pyrazinyle, pyridazinyle, triazinyle ou pyrimidinyle; les groupes phényle, phényloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₂₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène :
R₈ et R₉ représentent indépendamment l'un de l'autre un atome d'hydrogène ou un groupe C₁₋₆-alkyle,
   ou forment avec le ou les atomes qui les portent,
   dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
   dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane.

Les composés suivants, décrits dans le document EP1780210, sont exclus de la formule générale (I) :
- 2-(3-{[2-(4-chlorophenyl)-4-methylthiazol-5-yl]methoxycarbonylamino}piperidin-1-yl)benzoate de méthyle ;
- acide 2-(3-{[2-(4-chlorophenyl)-4-methylthiazol-5-yl]methoxycarbonylamino)piperidin-1-yl)benzoïque;
- 3-(3-{[2-(4-chlorophenyl)-4-methylthiazol-5-yl]methoxycarbonylamino}piperidin-1-yl)benzoate de méthyle ;
- acide 3-(3-{[2-(4-chlorophenyl)-4-methylthiazol-5-yl]methoxycarbonylamino}piperidin-1-yl)benzoïque.

On connaît du document FR 2 854 633 des dérivés de pipéridinyl- et pipérazinyl-alkylcarbonates qui présentent une activité inhibitrice de l'enzyme FAAH.

Parmi les composés de formule générale (I), un premier sous-groupe de composés est constitué des composés pour lesquels R₂ représente un atome d'hydrogène.

Parmi les composés de formule générale (I), un second sous-groupe de composés est constitué des composés pour lesquels n représente un nombre entier égal à 1 ou 2 et m représente un nombre entier égal à 2.

Parmi les composés de formule générale (I), un troisième sous-groupe de composés est constitué des composés pour lesquels A représente un groupe C₁₋₈₋alkylène, plus particulièrement un groupe méthylène ou éthylène.

Parmi les composés de formule générale (I), un quatrième sous-groupe de composés est constitué des composés pour lesquels R₁ représente un groupe R₅ éventuellement substitué par un ou plusieurs groupes R₆ et/ou R₇ ;
R₅ représente un groupe pyridinyle ou quinolinyle ;
R₆ représente un atome d'halogène, plus particulièrement un atome de chlore ou de fluor, un groupe cyano, -CH₂CN, C₁₋₆-alkyle, plus particulièrement méthyle, isopropyle, isobutyle, C₁₋₆-alcoxy, plus particulièrement méthoxy, éthoxy, C₁₋₆-haloalkyle, plus particulièrement trifluorométhyle, C₃₋₇-cycloalkyle, plus particulièrement cyclohexyle, C₃₋₇-cycloalkyle-C₁₋₃-alkylène-O-, plus particulièrement cyclopropyl-CH₂-O-, NR₈R₉, NR₈COR₉, NR₈CO₂R₉, NR₈SO₂R₉, CONR₈R₉, SO₂R₈ ou SO₂NR₈R₉;
R₇ représente un groupe choisi parmi un thiènyle, isoxazolyle, pyrazolyle, phényle, pyridinyle, pyrimidinyle, naphtalènyle, quinolinyle, isoquinolinyle, le ou les groupes R₇ pouvant être substitués par un ou plusieurs groupes R₆ identiques ou différents l'un de l'autre ;
R₈ et R₉ représentent indépendamment l'un de l'autre un atome d'hydrogène ou un groupe C₁₋₆-alkyle, plus particulièrement méthyle, éthyle, propyle, tert-butyle, ou forment avec le ou les atomes qui les portent un cycle choisi parmi les cycles pyrrolidine, pipéridine, morpholine.

Parmi les composés de formule générale (I), un cinquième sous-groupe de composés est constitué des composés pour lesquels R₃ représente un atome d'hydrogène, un groupe C₁₋₆-alkyle, plus particulièrement méthyle, ou un groupe trifluorométhyle.

Parmi les composés de formule générale (I), un sixième sous-groupe de composés est constitué des composés pour lesquels R₄ représente un thiazole éventuellement substitué par un ou plusieurs substituants choisis parmi un atome d'halogène, plus particulièrement de chlore, un groupe C₁₋₆-alkyle, plus particulièrement méthyle, C₁₋₆-haloalkyle, plus particulièrement trifluorométhyle, pyridinyle, CONR₈R₉; R₈ et R₉ représentent indépendamment l'un de l'autre un atome d'hydrogène ou un groupe C₁₋₆-alkyle, plus particulièrement un méthyle.

Parmi les composés de formule générale (I), un septième sous-groupe de composés est constitué par les composés de formule générale (I) dans laquelle à la fois R₁ et/ou R₂ et/ou R₃ et/ou R₄ et/ou n et/ou m et/ou A sont tels que définis dans les groupes ci-dessus.

Parmi les composés de formule générale (I), les composés suivants peuvent être cités (nomenclature IUPAC générée par le logiciel AutoNom) :
1. 6'-[Thiophèn-3-yl-3,4,5,6-tétrahydro-2H-[1,2']bipyridinyl-4-yl]-méthylcarbamate de thiazol-4-ylméthyle
2. 2-[(6'-Thiophèn-3-yl-3,4,5,6-tétrahydro-2H-[1,2']bipyridinyl-4-yl]-éthylcarbamate de thiazol-4-ylméthyle
3. 6'-[(4-Méthyl-thiophèn-3-yl)-3,4,5,6-tétrahydro-2H-[1,2']bipyridinyl-4-yl]-méthylcarbamate de thiazol-4-ylméthyle
4. 2-[6'-(4-Méthyl-thiophèn-3-yl)-3,4,5,6-tétrahydro-2H-[1,2']bipyridinyl-4-yl]-éthylcarbamate de thiazol-4-ylméthyle
5. 2-[6'-(5-Cyano-thiophèn-2-yl)-3,4,5,6-tétrahydro-2H-[1,2']bipyridinyl-4-yl]-éthylcarbamate de thiazol-4-ylméthyle
6. 2-[6'-(2-Méthyl-2H-pyrazol-3-yl)-3,4,5,6-tétrahydro-2H-[1,2']bipyridinyl-4-yl]-éthylcarbamate de thiazol-2-ylméthyle
7. 6'-[2-Méthyl-2H-pyrazol-3-yl)-3,4,5,6-tétrahydro-2H-[1,2']bipyridinyl-4-yl]-méthylcarbamate de thiazol-4-ylméthyle
8. 2-[6'-(2-Méthyl-2H-pyrazol-3-yl)-3,4,5,6-tétrahydro-2H-[1,2']bipyridinyl-4-yl]-éthylcarbamate de thiazol-4-ylméthyle
9. 2-[6'-(1-Méthyl-1H-pyrazol-4-yl)-3,4,5,6-tétrahydro-2H-[1,2']bipyridinyl-4-yl]-éthylcarbamate de thiazol-2-ylméthyle
10. 2-[6'-(1-Méthyl-1H-pyrazol-4-yl)-3,4,5,6-tétrahydro-2H-[1,2']bipyridinyl-4-yl]-éthylcarbamate de thiazol-4-ylméthyle
11. 2-[5'-(1-Méthyl-1H-pyrazol-4-yl)-3,4,5,6-tétrahydro-2H-[1,2']bipyridinyl-4-yl]-éthylcarbamate de thiazol-4-ylméthyle
12. 2-[5'-(2-Méthyl-2H-pyrazol-3-yl)-3,4,5,6-tétrahydro-2H-[1,2']bipyridinyl-4-yl]-éthylcarbamate de thiazol-4-ylméthyle
13. 2-[6'-(1-Isobutyl-1H-pyrazol-4-yl)-3,4,5,6-tétrahydro-2H-[1,2']bipyridinyl-4-yl]-éthylcarbamate de thiazol-2-ylméthyle
14. 6'-[(1-Isobutyl-1H-pyrazol-4-yl)-3,4,5,6-tétrahydro-2H-[1,2']bipyridinyl-4-yl]-méthylcarbamate de thiazol-4-ylméthyle
15. 2-[6'-(1-Isobutyl-1H-pyrazol-4-yl)-3,4,5,6-tétrahydro-2H-[1,2']bipyridinyl-4-yl]-éthylcarbamate de thiazol-4-ylméthyle
16. 2-[5'-(1-Isobutyl-1H-pyrazol-4-yl)-3,4,5,6-tétrahydro-2H-[1,2']bipyridinyl-4-yl]-éthylcarbamate de thiazol-4-ylméthyle
17. 6'-[(3,5-Diméthyl-isoxazol-4-yl)-3,4,5,6-tétrahydro-2H-[1,2']bipyridinyl-4-yl]-méthylcarbamate de thiazol-4-ylméthyle
18. 2-[6'-(3,5-Diméthyl-isoxazol-4-yl)-3,4,5,6-tétrahydro-2H-[1,2']bipyridinyl-4-yl]-éthylcarbamate de thiazol-4-ylméthyle
19. (4''-Méthoxy-3,4,5,6-tétrahydro-2H-[1,2';6',3'']terpyridin-4-yl)-méthylcarbamate de thiazol-4-ylméthyle
20. 2-(4''-Méthoxy-3,4,5,6-tétrahydro-2H-[1,2';6',3'']terpyridin-4-yl)-éthylcarbamate de thiazol-4-ylméthyle
21. 2-(5''-Fluoro-3,4,5,6-tétrahydro-2H-[1,2';6',3'']terpyridin-4-yl)-éthylcarbamate de thiazol-2-ylméthyle
22. 2-(5''-Fluoro-3,4,5,6-tétrahydro-2H-[1,2';6',3'']terpyridin-4-yl)-éthylcarbamate de thiazol-4-ylméthyle
23. 2-(5''-Fluoro-3,4,5,6-tétrahydro-2H-[1,2';5',3'']terpyridin-4-yl)-éthylcarbamate de thiazol-4-ylméthyle
24. 2-(6''-Ethoxy-3,4,5,6-tétrahydro-2H-[1,2';6',3'']terpyridin-4-yl)-éthylcarbamate de thiazol-4-ylméthyle
25. (6'-Pyrimidin-5-yl-3,4,5,6-tétrahydro-2H-[1,2']bipyridinyl-4-yl)-méthylcarbamate de thiazol-4-ylméthyle
26. {1-[5-(4-Fluoro-phényl)-pyridin-2-yl]-pyrrolidin-3-yl)}-méthylcarbamate de thiazol-2-ylméthyle
27. {1-[5-(4-Fluoro-phényl)-pyridin-2-yl]-pyrrolidin-3-yl}-méthylcarbamate de thiazol-4-ylméthyle
28. {2-[5'-(4-Fluoro-phényl)-3,4,5,6-tétrahydro-2H-[1,2']bipyridinyl-4-yl]}-éthylcarbamate de thiazol-2-ylméthyle
29. (+/-)-{2-[5'-(4-Fluoro-phényl)-3,4,5,6-tétrahydro-2H-[1,2']bipyridinyl-4-yl]}-éthylcarbamate de 2,2,2-trifluoro-1-thiazol-2-yl-éthyle
30. {2-[5'-(4-Fluoro-phényl)-3,4,5,6-tétrahydro-2H-[1,2']bipyridinyl-4-yl]}-éthylcarbamate de thiazol-4-ylméthyle
31. (+/-)-{2-[5'-(4-Fluoro-phényl)-3,4,5,6-tétrahydro-2H-[1,2']bipyridinyl-4-yl]}-éthylcarbamate de 2,2,2-trifluoro-1-thiazol-4-yl-éthyle
32. {2-[5'-(4-Fluoro-phényl)-3,4,5,6-tétrahydro-2H-[1,2']bipyridinyl-4-yl]}-éthylcarbamate de 2-méthyl-thiazol-4-ylméthyle
33. {2-[5'-(4-Fluoro-phényl)-3,4,5,6-tétrahydro-2H-[1,2']bipyridinyl-4-yl]}-éthylcarbamate de thiazol-5-ylméthyle
34. (+/-)-{2-[5'-(4-Fluoro-phényl)-3,4,5,6-tétrahydro-2H-[1,2']bipyridinyl-4-yl]}-éthylcarbamate de 2,2,2-trifluoro-1-thiazol-5-yl-éthyle
35. {2-[5'-(4-Chloro-phényl)-3,4,5,6-tétrahydro-2H-[1,2']bipyridinyl-4-yl]}-éthylcarbamate de thiazol-2-ylméthyle
36. {2-[5'-(4-Cyano-phényl)-3,4,5,6-tétrahydro-2H-[1,2']bipyridinyl-4-yl]}-éthylcarbamate de thiazol-2-ylméthyle
37. {2-[5'-(4-Cyano-phényl)-3,4,5,6-tétrahydro-2H-[1,2']bipyridinyl-4-yl]}-éthylcarbamate de thiazol-5-ylméthyle
38. {2-[5'-(4-Ethoxy-phényl)-3,4,5,6-tétrahydro-2H-[1,2']bipyridinyl-4-yl]}-éthylcarbamate de thiazol-2-ylméthyle
39. {2-[5'-(4-Ethoxy-phényl)-3,4,5,6-tétrahydro-2H-[1,2']bipyridinyl-4-yl]}-éthylcarbamate de thiazol-4-ylméthyl
40. {2-[5'-(4-Ethoxy-phényl)-3,4,5,6-tétrahydro-2H-[1,2']bipyridinyl-4-yl]}-éthylcarbamate de thiazol-5-ylméthyle
41. {2-[5'-(3-Cyano-phényl)-3,4,5,6-tétrahydro-2H-[1,2']bipyridinyl-4-yl]}-éthylcarbamate de thiazol-2-ylméthyle
42. {2-[5'-(3-Chloro-phényl)-3,4,5,6-tétrahydro-2H-[1,2']bipyridinyl-4-yl]}-éthylcarbamate de thiazol-2-ylméthyle
43. {2-[5'-(3-Carbamoyl-phényl)-3,4,5,6-tétrahydro-2H-[1,2']bipyridinyl-4-yl]}-éthylcarbamate de thiazol-4-ylméthyle
44. {2-[5'-(3-Fluoro-phényl)-3,4,5,6-tétrahydro-2H-[1,2']bipyridinyl-4-yl]}-éthylcarbamate de thiazol-2-ylméthyle
45. [6'-(3-Trifluorométhyl-phényl)-3,4,5,6-tétrahydro-2H-[1,2']bipyridinyl-4-yl]-méthylcarbamate de thiazol-4-ylméthyle
46. {2-[6'-(3-Trifluorométhyl-phényl)-3,4,5,6-tétrahydro-2H-[1,2']bipyridinyl-4-yl]}-éthylcarbamate de thiazol-4-ylméthyle
47. [6'-(4-Chloro-phényl)-3,4,5,6-tétrahydro-2H-[1,2']bipyridinyl-4-yl]-méthylcarbamate de thiazol-4-ylméthyle
48. {2-[6'-(4-Chloro-phényl)-3,4,5,6-tétrahydro-2H-[1,2']bipyridinyl-4-yl]}-éthylcarbamate de thiazol-4-ylméthyle
49. {2-[6'-(4-Fluoro-phényl)-3,4,5,6-tétrahydro-2H-[1,2']bipyridinyl-4-yl]}-éthylcarbamate de thiazol-2-ylméthyle
50. (+/-)-{2-[6'-(4-Fluoro-phényl)-3,4,5,6-tétrahydro-2H-[1,2']bipyridinyl-4-yl]}-éthylcarbamate de 2,2,2-trifluoro-1-thiazol-2-yl-éthyle
51. {2-[6'-(4-Fluoro-phényl)-3,4,5,6-tétrahydro-2H-[1,2']bipyridinyl-4-yl]}-éthylcarbamate de thiazol-4-ylméthyle
52. (+/-)-{2-[6'-(4-Fluoro-phényl)-3,4,5,6-tétrahydro-2H-[1,2']bipyridinyl-4-yl]}-éthylcarbamate de 2,2,2-trifluoro-1-thiazol-4-yl-éthyle
53. {2-[6'-(4-Fluoro-phényl)-3,4,5,6-tétrahydro-2H-[1,2']bipyridinyl-4-yl]}-éthylcarbamate de 2-méthyl-thiazol-4-ylméthyle
54. {2-[6'-(4-Fluoro-phényl)-3,4,5,6-tétrahydro-2H-[1,2']bipyridinyl-4-yl]}-éthylcarbamate de thiazol-5-ylméthyle
55. (+/-)-{2-[6'-(4-Fluoro-phényl)-3,4,5,6-tétrahydro-2H-[1,2']bipyridinyl-4-yl]}-éthylcarbamate de 2,2,2-trifluoro-1-thiazol-5-yl-éthyle
56. [6'-(4-Méthoxy-phényl)-3,4,5,6-tétrahydro-2H-[1,2']bipyridinyl-4-yl]-méthylcarbamate de thiazol-4-ylméthyle
57. {2-[6'-(4-Méthoxy-phényl)-3,4,5,6-tétrahydro-2H-[1,2']bipyridinyl-4-yl]}-éthylcarbamate de thiazol-4-ylméthyle
58. [6'-(3-Acétylamino-phényl)-3,4,5,6-tétrahydro-2H-[1,2']bipyridinyl-4-yl]-méthylcarbamate de thiazol-4-ylméthyle
59. {2-[6'-(3-Acétylamino-phényl)-3,4,5,6-tétrahydro-2H-[1,2']bipyridinyl-4-yl]}-éthylcarbamate de thiazol-4-ylméthyle
60. [6'-(3-Chloro-phényl)-3,4,5,6-tétrahydro-2H-[1,2']bipyridinyl-4-yl]-méthylcarbamate de thiazol-4-ylméthyle
61. {2-[6'-(3-Chloro-phényl)-3,4,5,6-tétrahydro-2H-[1,2']bipyridinyl-4-yl]}-éthylcarbamate de thiazol-4-ylméthyle
62. (6'-m-Tolyl-3,4,5,6-tétrahydro-2H-[1,2']bipyridinyl-4-yl)-méthylcarbamate de thiazol-4-ylméthyle
63. [2-(6'-o-Tolyl-3,4,5,6-tétrahydro-2H-[1,2']bipyridinyl-4-yl)]-éthylcarbamate de thiazol-4-ylméthyle
64. [6'-(2-Chloro-phényl)-3,4,5,6-tétrahydro-2H-[1,2']bipyridinyl-4-yl]-méthylcarbamate de thiazol-4-ylméthyle
65. [6'-(3,5-Difluoro-phényl)-3,4,5,6-tétrahydro-2H-[1,2']bipyridinyl-4-yl]-méthylcarbamate de thiazol-4-ylméthyle
66. {2-[6'-(3,5-Difluoro-phényl)-3,4,5,6-tétrahydro-2H-[1,2']bipyridinyl-4-yl]}-éthylcarbamate de thiazol-4-ylméthyle
67. [6'-(3,4,5-Triméthoxy-phényl)-3,4,5,6-tétrahydro-2H-[1,2']bipyridinyl-4-yl]-méthylcarbamate de thiazol-4-ylméthyle
68. {2-[6'-(2,4-Diméthoxy-phényl)-3,4,5,6-tétrahydro-2H-[1,2']bipyridinyl-4-yl]}-éthylcarbamate de thiazol-4-ylméthyle
69. [6'-(4-Isopropyl-phényl)-3,4,5,6-tétrahydro-2H-[1,2']bipyridinyl-4-yl]-méthylcarbamate de thiazol-4-ylméthyle
70. {2-[6'-(4-Isopropyl-phényl)-3,4,5,6-tétrahydro-2H-[1,2']bipyridinyl-4-yl]}-éthylcarbamate de thiazol-4-ylméthyle
71. [6'-(3-Isopropyl-phényl)-3,4,5,6-tétrahydro-2H-[1,2']bipyridinyl-4-yl]-méthylcarbamate de thiazol-4-ylméthyle
72. {2-[6'-(3-Isopropyl-phényl)-3,4,5,6-tétrahydro-2H-[1,2']bipyridinyl-4-yl]}-éthylcarbamate de thiazol-4-ylméthyle
73. [6'-(4-Cyclohexyl-phényl)-3,4,5,6-tétrahydro-2H-[1,2']bipyridinyl-4-yl]-méthylcarbamate de thiazol-4-ylméthyle
74. {2-[6'-(4-Cyclohexyl-phényl)-3,4,5,6-tétrahydro-2H-[1,2']bipyridinyl-4-yl]}-éthylcarbamate de thiazol-4-ylméthyle
75. [6'-(3-Diméthylcarbamoyl-phényl)-3,4,5,6-tétrahydro-2H-[1,2']bipyridinyl-4-yl]-méthylcarbamate de thiazol-4-ylméthyle
76. {2-[6'-(3-Diméthylcarbamoyl-phényl)-3,4,5,6-tétrahydro-2H-[1,2']bipyridinyl-4-yl]}-éthylcarbamate de thiazol-4-ylméthyle
77. {2-[6'-(4-Sulfamoyl-phényl)-3,4,5,6-tétrahydro-2H-[1,2']bipyridinyl-4-yl]}-éthylcarbamate de thiazol-4-ylméthyle
78. [6'-(4-Carbamoyl-phényl)-3,4,5,6-tétrahydro-2H-[1,2']bipyridinyl-4-yl]-méthylcarbamate de thiazol-4-ylméthyle
79. {2-[6'-(4-Carbamoyl-phényl)-3,4,5,6-tétrahydro-2H-[1,2']bipyridinyl-4-yl]}-éthylcarbamate de thiazol-4-ylméthyle
80. [6'-(3-Carbamoyl-phényl)-3,4,5,6-tétrahydro-2H-[1,2']bipyridinyl-4-yl]-méthylcarbamate de thiazol-4-ylméthyle
81. {2-[6'-(3-Carbamoyl-phényl)-3,4,5,6-tétrahydro-2H-[1,2']bipyridinyl-4-yl]}-éthylcarbamate de thiazol-4-ylméthyle
82. [6'-(3-Ethylcarbamoyl-phényl)-3,4,5,6-tetrahydro-2H-[1,2']bipyridinyl-4-yl]-méthylcarbamate de thiazol-4-ylméthyle
83. {2-[6'-(3-Ethylcarbamoyl-phényl)-3,4,5,6-tétrahydro-2H-[1,2']bipyridinyl-4-yl]}-éthylcarbamate de thiazol-4-ylméthyle
84. [6'-(3-Propylcarbamoyl-phényl)-3,4,5,6-tétrahydro-2H-[1,2']bipyridinyl-4-yl]-méthylcarbamate de thiazol-4-ylméthyle
85. {2-[6'-(4-Méthanesulfonylamino-phényl)-3,4,5,6-tétrahydro-2H-[1,2']bipyridinyl-4-yl]}-éthylcarbamate de thiazol-4-ylméthyle
86. 4-{4-[(Thiazol-4-ylméthoxycarbonylamino)-méthyl]-3,4,5,6-tétrahydro-2H-[1,2']bipyridinyl-6'-yl}-phénylcarbamate de méthyle
87. 4-{4-[2-(Thiazol-4-ylméthoxycarbonylamino)-éthyl]-3,4,5,6-tetrahydro-2H-[1,2']bipyridinyl-6'-yl}-phénylcarbamate de méthyle
88. {2-[6'-(3-Pyrrolidin-1-yl-phényl)-3,4,5,6-tétrahydro-2H-[1,2']bipyridinyl-4-yl]}-éthylcarbamate de thiazol-4-ylméthyle
89. [6'-(2-Morpholin-4-yl-phényl)-3,4,5,6-tétrahydro-2H-[1,2']bipyridinyl-4-yl]-méthylcarbamate de thiazol-4-ylméthyle
90. {2-[6'-(2-Morpholin-4-yl-phényl)-3,4,5,6-tétrahydro-2H-[1,2']bipyridinyl-4-yl]}-éthylcarbamate de thiazol-4-ylméthyle
91. {2-[6'-(3-Ethoxy-phényl)-3,4,5,6-tétrahydro-2H-[1,2']bipyridinyl-4-yl]}-éthylcarbamate de thiazol-4-ylméthyle
92. {2-[6'-(4-Cyclopropylméthoxy-phényl)-3,4,5,6-tétrahydro-2H-[1,2']bipyridinyl-4-yl]}-éthylcarbamate de thiazol-4-ylméthyle
93. [6'-(3-Cyclopropylméthoxy-phényl)-3,4,5,6-tétrahydro-2H-[1,2']bipyridinyl-4-yl]-méthylcarbamate de thiazol-4-ylméthyle
94. {2-[6'-(3-Cyclopropylméthoxy-phényl)-3,4,5,6-tétrahydro-2H-[1,2']bipyridinyl-4-yl]}-éthylcarbamate de thiazol-4-ylméthyle
95. [6'-(4-Cyanométhyl-phényl)-3,4,5,6-tétrahydro-2H-[1,2']bipyridinyl-4-yl]-méthylcarbamate de thiazol-4-ylméthyle
96. [6'-(3-Cyanométhyl-phényl)-3,4,5,6-tétrahydro-2H-[1,2']bipyridinyl-4-yl]-méthylcarbamate de thiazol-4-ylméthyle
97. {6'-[4-(Pipéridine-1-sulfonyl)-phényl]-3,4,5,6-tétrahydro-2H-[1,2']bipyridinyl-4-yl]}-méthylcarbamate de thiazol-4-ylméthyle
98. (2-{6'-[4-(Pipéridine-1-sulfonyl)-phényl]-3,4,5,6-tétrahydro-2H-[1,2']bipyridinyl-4-yl})-éthylcarbamate de thiazol-4-ylméthyle
99. [6'-(4-Acétylamino-phényl)-3,4,5,6-tétrahydro-2H-[1,2']bipyridinyl-4-yl]-méthylcarbamate de thiazol-4-ylméthyle
100. {2-[6'-(4-Acétylamino-phényl)-3,4,5,6-tétrahydro-2H-[1,2']bipyridinyl-4-yl]}-éthylcarbamate de thiazol-4-ylméthyle
101. {2-[6'-(3-Méthanesulfonyl-phényl)-3,4,5,6-tétrahydro-2H-[1,2']bipyridinyl-4-yl]}-éthylcarbamate de thiazol-4-ylméthyle
102. [6'-(3-Ethanesulfonyl-phényl)-3,4,5,6-tétrahydro-2H-[1,2']bipyridinyl-4-yl]-méthylcarbamate de thiazol-4-ylméthyle
103. {2-[6'-(3-Ethanesulfonyl-phényl)-3,4,5,6-tétrahydro-2H-[1,2']bipyridinyl-4-yl]}-éthylcarbamate de thiazol-4-ylméthyle
104. {2-[1-(6-Chloro-quinolin-2-yl)-pipéridin-4-yl]}-éthylcarbamate de thiazol-2-ylméthyle
105. {2-[1-(6-Chloro-quinolin-2-yl)-pipéridin-4-yl]}-éthylcarbamate de 4-trifluorométhyl-thiazol-2-ylméthyle
106. (+/-)-{2-[1-(6-Chloro-quinolin-2-yl)-pipéridin-4-yl]}-éthylcarbamate de 2,2,2-trifluoro-1-thiazol-2-yl-éthyle
107. (+/-)-{2-[1-(6-Chloro-quinolin-2-yl)-pipéridin-4-yl]}-éthylcarbamate de 1-thiazol-4-yl-éthyle
108. (+/-)-{2-[1-(6-Chloro-quinolin-2-yl)-pipéridin-4-yl]}-éthylcarbamate de 2,2,2-trifluoro-1-thiazol-4-yl-éthyle
109. {2-[1-(6-Chloro-quinolin-2-yl)-pipéridin-4-yl]}-éthylcarbamate de thiazol-4-ylméthyle
110. {2-[1-(6-Chloro-quinolin-2-yl)-pipéridin-4-yl]}-éthylcarbamate de 2-méthyl-thiazol-4-ylméthyle
111. {2-[1-(6-Chloro-quinolin-2-yl)-pipéridin-4-yl]}-éthylcarbamate de 2-trifluorométhyl-thiazol-4-ylméthyle
112. {2-[1-(6-Chloro-quinolin-2-yl)-pipéridin-4-yl]}-éthylcarbamate de 2-chloro-thiazol-4-ylméthyle
113. {2-[1-(6-Chloro-quinolin-2-yl)-pipéridin-4-yl]}-éthylcarbamate de 2-pyridin-3-yl-thiazol-4-ylméthyle
114. {2-[1-(6-Chloro-quinolin-2-yl)-pipéridin-4-yl]}-éthylcarbamate de 2-pyridin-4-yl-thiazol-5-ylméthyle
115. {2-[1-(6-Chloro-quinolin-2-yl)-pipéridin-4-yl]}-éthylcarbamate de thiazol-5-ylméthyle
116. (+/-)-{2-[1-(6-Chloro-quinolin-2-yl)-pipéridin-4-yl]}-éthylcarbamate de 2,2,2-trifluoro-1-thiazol-5-yl-éthyle
117. {2-[1-(6-Fluoro-quinolin-2-yl)-pipéridin-4-yl]}-éthylcarbamate de thiazol-5-ylméthyle
118. {2-[1-(7-Fluoro-quinolin-2-yl)-pipéridin-4-yl]}-éthylcarbamate de thiazol-5-ylméthyle
119. (6'-Naphthalèn-1-yl-3,4,5,6-tétrahydro-2H-[1,2']bipyridinyl-4-yl)-méthylcarbamate de thiazol-4-ylméthyle
120. [2-(6'-Naphthalèn-1-yl-3,4,5,6-tétrahydro-2H-[1,2']bipyridinyl-4-yl)]-éthylcarbamate de thiazol-4-ylméthyle
121. (6'-Naphthalèn-2-yl-3,4,5,6-tétrahydro-2H-[1,2']bipyridinyl-4-yl)-méthylcarbamate de thiazol-4-ylméthyle
122. (6'-Naphthalèn-2-yl-3,4,5,6-tétrahydro-2H-[1,2']bipyridinyl-4-yl)-éthylcarbamate de thiazol-4-ylméthyle
123. (6'-Quinolin-4-yl-3,4,5,6-tétrahydro-2H-[1,2']bipyridinyl-4-yl)-méthylcarbamate de thiazol-4-ylméthyle
124. [2-(6'-Quinolin-4-yl-3,4,5,6-tétrahydro-2H-[1,2']bipyridinyl-4-yl)]-éthylcarbamate de thiazol-4-ylméthyle
125. (6'-Quinolin-6-yl-3,4,5,6-tétrahydro-2H-[1,2']bipyridinyl-4-yl)-méthylcarbamate de thiazol-4-ylméthyle
126. [2-(6'-Quinolin-6-yl-3,4,5,6-tétrahydro-2H-[1,2']bipyridinyl-4-yl)]-éthylcarbamate de thiazol-4-ylméthyle
127. (6'-Isoquinolin-4-yl-3,4,5,6-tétrahydro-2H-[1,2']bipyridinyl-4-yl)-méthylcarbamate de thiazol-4-ylmethyle
128. [2-(6'-Isoquinolin-4-yl-3,4,5,6-tétrahydro-2H-[1,2']bipyridinyl-4-yl)]-éthylcarbamate de thiazol-4-ylméthyle
129. (6'-Isoquinolin-5-yl-3,4,5,6-tétrahydro-2H-[1,2']bipyridinyl-4-yl)-méthylcarbamate de thiazol-4-ylméthyle
130. (6'-Isoquinolin-5-yl-3,4,5,6-tétrahydro-2H-[1,2']bipyridinyl-4-yl)-éthylcarbamate de thiazol-4-ylméthyle
131. 2-[5'-(4-Fluoro-phényl)-3,4,5,6-tétrahydro-2H-[1,2']bipyridinyl-4-yl]-éthylcarbamate de 2-carbamoyl-thiazol-4-ylméthyle
132. 2-[5'-(4-Fluoro-phényl)-3,4,5,6-tétrahydro-2H-[1,2']bipyridinyl-4-yl]-éthylcarbamate de 2-methylcarbamoyl-thiazol-4-ylméthyle
133. 2-[5'-(4-Fluoro-phényl)-3,4,5,6-tétrahydro-2H-[1,2']bipyridinyl-4-yl]-éthylcarbamate de 4-carbamoyl-thiazol-2-ylméthyle
134. 2-[5'-(4-Fluoro-phényl)-3,4,5,6-tétrahydro-2H-[1,2']bipyridinyl-4-yl]-éthylcarbamate de 4-méthylcarbamoyl-thiazol-2-ylméthyle
à l'état de base ou de sel d'addition à un acide.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis (Z)* ou *trans (E)*. Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tert-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode ;
- « (+/-) » désigne un composé sous forme de mélange racémique.

Les composés de l'invention peuvent être préparés selon différentes méthodes, illustrées par les schémas qui suivent.

Ainsi une première méthode (schéma 1) consiste à faire réagir une amine de formule générale (II), dans laquelle A, R₁, R₂, m et n sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

Une variante d'obtention des composés de formule générale (I) (schéma 1) consiste à faire réagir une amine de formule générale (II), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IV), dans laquelle A, R₁, R₂, m et n sont tels que définis dans la formule générale (I) définie ci-dessus, et Z représente un atome d'hydrogène ou un groupe nitro. Le dérivé carbamate de formule générale (IV) ainsi obtenu est ensuite transformé en composé de formule générale (I), par action d'un alcool de formule générale HOCHR₃R₄ (IIIa), dans laquelle R₃ et R₄ sont tels que définis dans la formule générale (I) définie ci-dessus, en présence d'une base telle que la triéthylamine, la pyridine, la *N,N-*diméthylaminopyridine ou la diisopropyléthylamine, dans un solvant tel que le toluène ou le dichloroéthane, à une température comprise entre la température ambiante et la température de reflux du solvant.

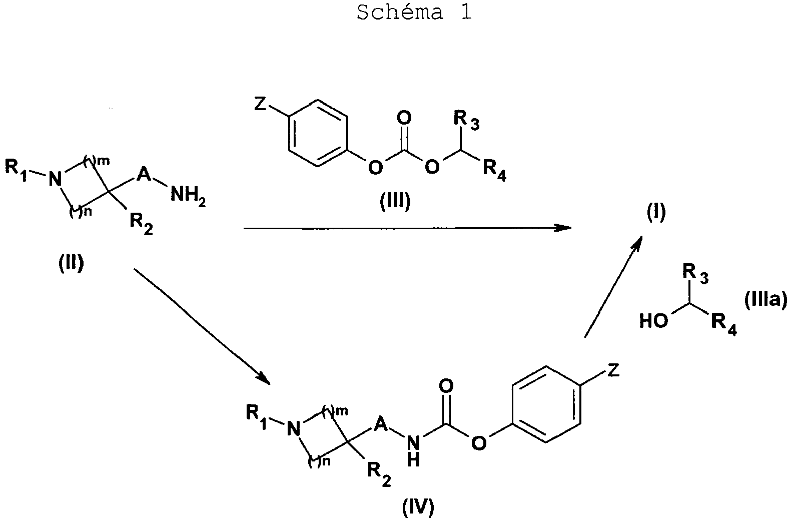

Une seconde méthode (schéma 2) consiste à faire réagir dans un premier temps une amine de formule générale (IIa), dans laquelle A, R₂, m et n sont tels que définis dans la formule générale (I) définie ci-dessus, et PG représente un groupe protecteur tel qu'un Boc (tert-butyloxycarbonyl), un Cbz (benzyloxycarbonyl), un benzyle ou un benzhydrile, avec un carbonate de formule générale (III) telle que définie ci-dessus, dans les conditions décrites ci-dessus lors de la réaction de l'amine de formule générale (II) avec le carbonate de formule générale (III), suivie d'une réaction de déprotection, par exemple en présence d'une solution d'acide chlorhydrique (5N) dans l'isopropanol ou le dioxane, pour obtenir l'intermédiaire de formule générale (Ia), dans laquelle A, R₂, R₃, R₄, m et n sont tels que définis dans la formule générale (I).
Une variante d'obtention des intermédiaires de formule générale (Ia) (schéma 2) consiste à faire réagir une amine de formule générale (IIa), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IVa), dans laquelle A, R₂, m et n sont tels que définis dans la formule générale (I) définie ci-dessus, PG est tel que défini ci-dessus et Z représente un atome d'hydrogène ou un groupe nitro. Le dérivé carbamate de formule générale (IVa) ainsi obtenu est ensuite transformé en composé de formule générale (Ia), par action d'un alcool de formule générale HOCHR₃R₄ (IIIa), telle que définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant, suivie par une réaction de déprotection, par exemple en présence d'une solution d'acide chlorhydrique (5N) dans l'isopropanol ou le dioxane.
Le composé de formule générale (I) est ensuite obtenu par réaction du composé de formule générale (Ia) avec un dérivé de formule générale (V), dans laquelle R₁ est tel que défini dans la formule générale (I) et U₁ représente un atome d'halogène ou un groupe O-triflate, en utilisant les conditions de réactions de substitution nucléophile aromatique ou hétéroaromatique, par exemple au moyen d'une base telle que la triéthylamine, la diisopropyléthylamine, la pyridine ou la *N,N*-diméthylaminopyridine dans un solvant tel que le dichlorométhane, le dichloroéthane, l'acétonitrile, la *N,N*-diméthylformamide, le dioxane ou le tétrahydrofurane, à une température comprise entre 0°C et la température de reflux du solvant. Cette transformation peut être également réalisée en utilisant les conditions de *N-*arylation ou *N*-hétéroarylation de Buchwald, par exemple au moyen d'un catalyseur au Palladium ou au Cuivre.

Selon le schéma 2, les composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus, peuvent être également préparés selon une réaction de couplage, catalysée au moyen d'un métal de transition, par exemple le Palladium (0), réalisée sur le composé de formule générale (Ib), dans laquelle A, R₂, R₃, R₄, R₅, m et n sont tels que définis dans la formule générale (I) et U₂ représente un atome de chlore, de brome, d'iode ou un groupement triflate, U₂ étant dans la position où l'on souhaite introduire le groupe R₆ ou R₇ (schéma 2) :
soit par une réaction de type Suzuki, par exemple au moyen d'un acide boronique d'alkyle, de cycloalkyle, d'aryle ou d'hétéroaryle,
soit selon une réaction de type Stille, par exemple en utilisant un dérivé *tri*-alkylstanneux d'aryle ou d'hétéroaryle soit par une réaction de type Négishi, par exemple en utilisant un dérivé zincate d'halogénure d'alkyle, de cycloalkyle, d'aryle ou d'hétéroaryle.
L'intermédiaire de formule générale (Ib) telle que définie ci-dessus est préalablement obtenu en faisant réagir une amine de formule générale (Ia) telle que définie ci-dessus avec un dérivé de formule générale (Va) dans laquelle R₅, U₁ et U₂ sont tels que définis ci-dessus en utilisant les réactions de substitution nucléophile aromatique, hétéroaromatique ou de *N*-arylation, *N*-hétéroarylation de Buchwald, par exemple au moyen d'un catalyseur au Palladium ou au Cuivre.

Une variante d'obtention des intermédiaires de formule générale (Ib) (schéma 2) consiste à faire réagir dans un premier temps une amine de formule générale (IIb), dans laquelle A, R₅, R₂, m et n sont tels que définis dans la formule générale (I) définie ci-dessus, et U₂ est tel que défini ci-dessus, avec un carbonate de formule générale (III) telle que définie ci-dessus, dans les conditions décrites ci-dessus lors de la réaction de l'amine de formule générale (II) avec le carbonate de formule générale (III), pour obtenir l'intermédiaire de formule générale (Ib), dans laquelle A, R₅, R₂, R₃, R₄, m et n sont tels que définis dans la formule générale (I), et U₂ est tel que défini ci-dessus.

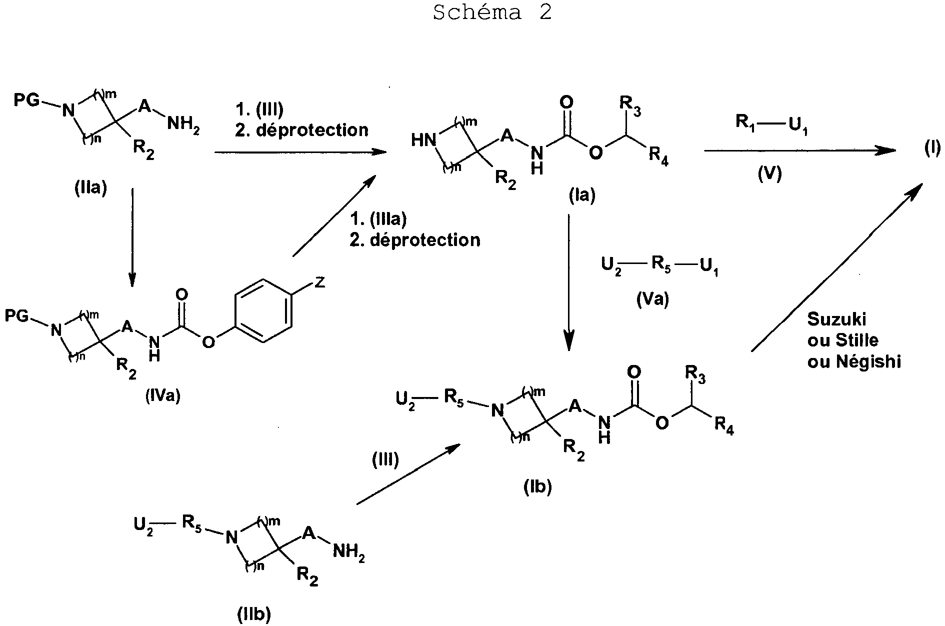

Les composés de formules générales (II), (IIa), (IIb), (III), (IIIa), (V) et (Va) ainsi que les autres réactifs sont disponibles dans le commerce ou décrits dans la littérature, ou bien peuvent être préparés selon des méthodes qui y sont décrites ou qui sont connues de l'homme du métier. Notamment, le carbonate de formule générale (III) peut être préparé selon toute méthode décrite dans la littérature, par exemple par réaction d'un alcool de formule générale HOCHR₃R₄ (IIIa), dans laquelle R₃ et R₄ sont tels que définis dans la formule générale (I) telle que définie ci-dessus, avec le chloroformiate de phényle ou de 4-nitrophényle, en présence d'une base telle que la triéthylamine, la *N*-méthylmorpholine ou la diisopropyléthylamine, dans un solvant tel que le dichlorométhane ou le tétrahydrofurane, à une température comprise entre 0°C et la température ambiante.

Les exemples qui suivent illustrent la préparation de quelques composés de l'invention. Ces exemples ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius. Les numéros indiqués entre parenthèses dans les titres des exemples correspondent à ceux de la 1ère colonne du tableau ci-après.

La nomenclature UICPA (Union Internationale de Chimie Pure et Appliquée - IUPAC en anglais) a été utilisée pour la dénomination des composés dans les exemples ci-dessous.

### Exemple 1 (Composé N°30)

### 2-[5'-(4-Fluoro-phényl)-3,4,5,6-tétrahydro-2H-[1,2']bipyridinyl-4-yl]-éthylcarbamate de thiazol-4-ylméthyle

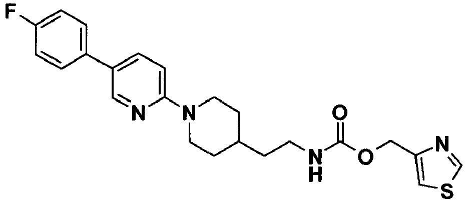

### 1.1. 2-(5'-Bromo-3,4,5,6-tétrahydro-2H-[1,2']bipyridinyl-4-yl)-éthanol

Dans un autoclave, on introduit 11 g (46,43 mmoles) de 2,5-dibromopyridine, 6 g (46,43 mmoles) de pipéridin-4-yl-éthanol et 6,74 g (48,76 mmoles) de carbonate de potassium dans 8 ml de DMSO. On chauffe ensuite à 160°C pendant 20 heures.
On laisse revenir à température ambiante puis on reprend le mélange réactionnel par de l'acétate d'éthyle et de l'eau. On sépare la phase aqueuse, on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite.
On obtient ainsi 11 g de produit sous forme d'huile utilisé tel quel dans l'étape suivante.

### 1.2. 2-[5'-(4-Fluoro-phényl)-3,4,5,6-tétrahydro-2H-[1,2']bipyridinyl-4-yl]-éthanol

Sous atmosphère inerte, on introduit 3,6 g (12,62 mmoles) de 2-(5'-Bromo-3,4,5,6-tétrahydro-2H-[1,2']bipyridinyl-4-yl)-éthanol, préparé à l'étape 1.1., 3,53 g (25,25 mmoles) de l'acide 4-fluorophénylboronique, 5,23 g (37,87 mmoles) de carbonate de potassium et 4,88 g (15,15 mmoles) de bromure de tétrabutylammonium en suspension dans 20 ml d'eau. On ajoute ensuite 0,142 g (0,63 mmole) de Pd(OAc)₂. On porte ensuite le mélange réactionnel au reflux pendant 24 heures.
On laisse revenir à température ambiante, on sépare les sels par filtration sur célite, puis on reprend le filtrat avec de l'acétate d'éthyle, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 50/50 d'acétate d'éthyle et de cyclohexane.
On obtient ainsi 1,6 g de produit sous forme de poudre blanche.
PF (°C) = 118-120°C

### 1.3. 2-{2-[5'-(4-Fluoro-phényl)-3,4,5,6-tétrahydro-2H-[1,2']bipyridinyl-4-yl]-éthyl}-isoindole-1,3-dione

A une solution de 2 g (6,66 mmoles) de 2-[5'-(4-Fluorophényl)-3,4,5,6-tétrahydro-2H-[1,2']bipyridinyl-4-yl]-éthanol, préparé à l'étape 1.2., de 2,096 g (7,99 mmoles) de triphénylphosphine et de 1,077 g (7,32 mmoles) de phtalimide dans 40 ml de tétrahydrofurane, refroidie à environ -2°C, on ajoute goutte à goutte, sous atmosphère inerte, une solution de 1,61 g (7,99 mmoles) de diisopropylazodicarboxylate (DIAD) dans 4 ml de tétrahydrofurane tout en maintenant la température du milieu réactionnel entre -2°C et 0°C. On poursuit l'agitation à 0°C pendant 1 heure, puis à température ambiante pendant 12 heures.
On concentre sous pression réduite, on reprend le résidu avec du dichlorométhane et de l'eau. On sépare la phase aqueuse puis on l'extrait 2 fois avec du dichlorométhane. On réunit les phases organiques et on les lave successivement avec une solution aqueuse d'acide chlorhydrique (1N), puis une solution aqueuse saturée d'hydrogénocarbonate de sodium et une solution aqueuse saturée en chlorure de sodium. On sèche la phase organique sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.
On obtient ainsi 2,1 g du produit attendu sous forme de poudre blanche.
PF (°C) = 180-182°C

### 1.4. 2-[5'-(4-Fluoro-phényl)-3,4,5,6-tétrahydro-2H-[1,2']bipyridinyl-4-yl]-éthylamine

A une solution de 1,3 g (3,03 mmoles) de 2-{2-[5'-(4-Fluorophényl)-3,4,5,6-tétrahydro-2H-[1,2']bipyridinyl-4-yl]-éthyl}-isoindole-1,3-dione, préparé à l'étape 1.3., dans 30 ml d'éthanol, on ajoute lentement à température ambiante 0,485 g (15,13 mmoles) d'hydrazine monohydrate. On porte ensuite le mélange réactionnel au reflux pendant trois heures.
On laisse revenir à température ambiante, on sépare l'insoluble par filtration et on concentre le filtrat sous pression réduite. On reprend le résidu par 20 ml d'éther et on laisse sous agitation à température ambiante pendant une heure. On sépare de nouveau l'insoluble et on concentre le filtrat sous pression réduite.
On obtient ainsi 0,70 g du produit attendu sous forme de poudre blanche.
PF (°C) = 88-94°C
RMN ¹H (CDCl₃) δ (ppm) : 8,3 (d, 1H) ; 7,55 (dd, 1H) ; 7,35 (m, 2H) ; 7,05 (d, 1H) ; 7,1 (d, 1H) ; 6,65 (d, 1H) ; 4,25 (large d, 2H) ; 3,0-2,8 (m, 4H) ; 1,8 (m, 2H) ; 1,6-1,1 (m, 5H).

### 1.5. 2-[5'-(4-Fluoro-phényl)-3,4,5,6-tétrahydro-2H-[1,2']bipyridinyl-4-yl]-éthylcarbamate de thiazol-4-ylméthyle

On chauffe à 70°C pendant 12 heures, une solution de 0,3 g (1,07 mmoles) de 2-[5'-(4-fluoro-phényl)-3,4,5,6-tétrahydro-2H-[1,2']bipyridinyl-4-yl]-éthylamine, préparé à l'étape 1.4., de 0,35 g (1,18 mmoles) de (4-nitro-phényl)-carbonate de thiazol-4-ylméthyle (W02008/013834) et de 0,21 g (1,61 mmoles) de N,N-diisopropyléthylamine dans 5 ml de 1,2-dichloroéthane.
On laisse revenir à température ambiante, on sépare l'insoluble par filtration et on concentre le filtrat sous pression réduite. On reprend le résidu par du dichlorométhane et de l'eau, on sépare la phase aqueuse, on l'extrait trois fois avec du dichlorométhane, on lave les phases organiques réunies avec une solution aqueuse saturée en chlorure de sodium et on les sèche sur sulfate de sodium. Après évaporation du solvant, on purifie le résidu obtenu par chromatographie sur gel de silice en éluant avec un mélange 95/5 de dichlorométhane et de méthanol.
On obtient ainsi 0,3 g de produit pur sous forme de poudre blanche.
LC-MS : M+H = 441
PF(°C) : 130-132°C
RMN ¹H (DMSO) δ (ppm) : 9,1 (s, 1H) ; 8,45 (s, 1H) ; 7,85 (d, 1H) ; 7,7 (m, 4H) ; 7,3 (m, 2H) ; 6,95 (d, 1H); 5,15 (s, 2H) ; 4,30 (large d, 2H); 3,1 (m, 2H) ; 2,8 (m, 2H); 1,8 (m, 2H) ; 1, 6 (m, 1H) ; 1,4 (m, 2H) ; 1, 1 (m, 2H).

### Exemple 2 (Composé N°28)

### 2-[5'-(4-Fluoro-phényl)-3,4,5,6-tétrahydro-2H-[1,2']bipyridinyl-4-yl]-éthylcarbamate de thiazol-2-ylméthyle

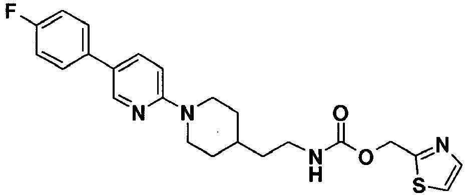

On procède suivant le mode opératoire décrit dans l'exemple 1 (étape 1.4.). A partir de 0,3 g (1,07 mmoles) de 2-[5'-(4-fluoro-phényl)-3,4,5,6-tétrahydro-2H-[1,2']bipyridinyl-4-yl]-éthylamine, décrit dans l'exemple 1 (étape 1.3.), de 0,35 g (1,18 mmoles) de (4-nitro-phényl)-carbonate de thiazol-2-ylméthyle (EP486948A2), et après chromatographie sur gel de silice en éluant avec un mélange 98/2 de dichlorométhane et de méthanol, on obtient 0,25 g de produit pur sous forme de poudre blanche.
LC-MS : M+H = 441
PF(°C) : 131-133°C
RMN ¹H (DMSO) δ (ppm) : 8,45 (s, 1H); 7,80 (m, 3H); 7,65 (dd, 2H) ; 7,45 (m, 1H) ; 7,25 (dd, 2H) ; 6,9 (d, 1H); 5,30 (s, 2H) ; 4,30 (large d, 2H) ; 3,1 (m, 2H) ; 2,8 (m, 2H) ; 1,75 (m, 2H) ; 1,6 (m, 1H); 1,4 (m, 2H) ; 1,1 (m, 2H).

### Exemple 3 (Composé N°33)

### 2-[5'-(4-Fluoro-phényl)-3,4,5,6-tétrahydro-2H-[1,2']bipyridinyl-4-yl]-éthylcarbamate de thiazol-5-ylméthyle

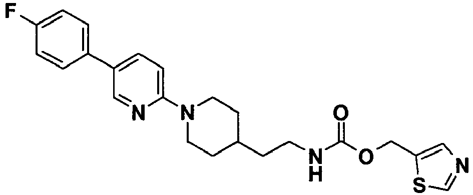

On procède suivant le mode opératoire décrit dans l'exemple 1 (étape 1.4.). A partir de 0,16 g (0,53 mmole) de 2-[5'-(4-fluoro-phényl)-3,4,5,6-tétrahydro-2H-[1,2']bipyridinyl-4-yl]-éthylamine, décrit dans l'exemple 1 (étape 1.3.), de 0,22 g (0,8 mmole) (4-nitro-phényl)-carbonate de thiazol-5-ylméthyle, et après chromatographie sur gel de silice en éluant avec un mélange 40/60 d'acétate d'éthyle et de cyclohexane, on obtient 0,180 g de produit pur sous forme de poudre blanche.
LC-MS : M+H = 441
PF(°C) : 100-102°C
RMN ¹H (DMSO) δ (ppm) : 9,1 (s, 1H) ; 8,4 (large s, 1H); 7,9 (s, 1H) ; 7,8 (dd, 1H); 7,60 (dd, 2H) ; 7,20 (m, 3H); 6,90 (d, 1H) ; 5,2 (s, 2H); 4,30 (large d, 2H) ; 3 (m, 2H); 2,75 (m, 2H) ; 1,8-1,1 (m, 7H).

### Exemple 4 (Composé N°50)

### (+/-)-2-[6'-(4-Fluoro-phényl)-3,4,5,6-tétrahydro-2H-[1,2']bipyridinyl-4-yl]-éthylcarbamate de 2,2,2-trifluoro-1-thiazol-2-yléthyle

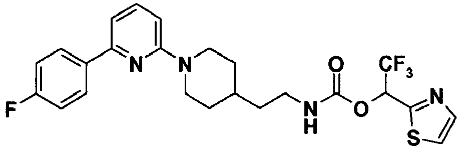

### 4.1. 2-[6'-(4-fluoro-phényl)-3,4,5,6-tétrahydro-2H-[1,2']bipyridinyl-4-yl]-éthanol

Sous atmosphère inerte, on introduit 3,6 g (12,62 mmoles) de 2-(6'-bromo-3,4,5,6-tétrahydro-2H-[1,2']bipyridinyl-4-yl)-éthanol (WO2004/099176), 3,53 g (25,25 mmoles) de l'acide 4-fluorophénylboronique, 5,23 g (37,87 mmoles) de carbonate de potassium et 4,88 g (15,15 mmoles) de bromure de tétrabutylammonium en suspension dans 20 ml d'eau. On ajoute ensuite 0,142 g (0,63 mmole) de Pd (OAc)₂. On porte ensuite le mélange réactionnel au reflux pendant 24 heures.
On laisse revenir à température ambiante, on sépare les sels par filtration sur célite, puis on reprend filtrat avec de l'acétate d'éthyle, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 99/1 de dichlorométhane et de méthanol.
On obtient 3,6 g de produit sous forme de poudre blanche.
PF (°C) = 96-100°C

### 4.2. 2-{2-[6'-(4-Fluoro-phényl)-3,4,5,6-tétrahydro-2H-[1,2']bipyridinyl-4-yl]-éthyl}-isoindole-1,3-dione

On procède suivant le mode opératoire décrit dans l'exemple 1 (étape 1.2.). A partir de 2 g (6,66 mmoles) de 2-[6'-(4-fluoro-phényl)-3,4,5,6-tétrahydro-2H-[1,2']bipyridinyl-4-yl]-éthanol, préparé à l'étape 4.1., de 2,096 g (7,99 mmomles) de triphénylphosphine, de 1,077 g (7,32 mmoles) de phtalimide, de 1,61 g (7,99 mmoles) de diisopropylazodicarboxylate (DIAD), et après chromatographie sur gel de silice en éluant avec un mélange 15/85 d'acétate d'éthyle et de cyclohexane, on obtient 1,4 g de produit pur sous forme de poudre blanche.
PF (°C) = 112-114°C

### 4.3. 2-[6'-(4-Fluoro-phényl)-3,4,5,6-tétrahydro-2H-[1,2']bipyridinyl-4-yl]-éthylamine

On procède suivant le mode opératoire décrit dans l'exemple 1 (étape 1.3.). A partir de 1,3 g (3,03 mmoles) de 2-{2-[6'-(4-fluoro-phényl)-3,4,5,6-tétrahydro-2H-[1,2']bipyridinyl-4-yl]-éthyl}-isoindole-1,3-dione, préparé à l'étape 4.2. et de 0,485 g (15,13 mmoles) d'hydrazine monohydrate, on obtient 0,8 g de produit sous forme d'un liquide incolore utilisé tel quel dans l'étape suivante.
RMN ¹H (CDCl₃) δ (ppm) : 8,2 (d, 1H) ; 8,15 (d, 1H) ; 7,75 (dd, 1H) ; 7,35 (large t, 2H) ; 7,2 (d, 1H) ; 6,75 (d, 1H) ; 4,75 (large d, 2H) ; 3,2-3,0 (m, 4H) ; 2,15 (m, 2H) ; 1,8-1,3 (m, 5H).

### 4.4. 2-[6'-(4-Fluoro-phényl)-3,4,5,6-tétrahydro-2H-[1,2']bipyridinyl-4-yl]-éthylcarbamate de 4-nitro-phényle

A une solution de de 5 g (16,7 mmoles) de 2-[6'-(4-fluorophényl)-3,4,5,6-tétrahydro-2H-[1,2']bipyridinyl-4-yl]-éthylamine, préparé à l'étape 4.3., de 4,32 g (33,40 mmoles) de *N*,*N*-diisopropyléthylamine et de 0,10 g (0,84 mmole de *N,N-*diméthylaminopyridine dans 80 ml de dichlorométhane, refroidie à environ 0°C, on ajoute par petites portions 3,7 g (18,37 mmoles) de chloroformiate de 4-nitrophényle. On poursuit l'agitation à 0°C pendant 1 heure puis à température ambiante pendant 2 heures.
On ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite.
Après chromatographie sur gel de silice en éluant avec un mélange 98/2 de dichlorométhane et de méthanol, on obtient 4,6 g de produit pur sous forme de poudre blanche.
PF(°C) : 138-142°C

### 4.5. 2,2,2-Trifluoro-1-thiazol-2-yl-éthanol

A une solution de 2 g (17,68 mmoles) de thiazole-2-carboxaldéhyde, de 0,88 ml (0,88 mmole) d'une solution 1M de fluorure de tétrabutylammonium dans le THF, dans 88 ml de THF, on ajoute lentement à environ 0° (bain de glace) 2,7 g (19,44 mmoles) de trifluorométhyltriméthylsilane (TMS-CF₃). On poursuit l'agitation à température ambiante pendant 2 heures. On ajoute au milieu réactionnel 25 ml d'une solution aqueuse d'acide chlorhydrique 1N et de l'acétate d'éthyle. On sépare la phase aqueuse, on l'extrait deux fois avec de l'acétate d'éthyle, on lave successivement les phases organiques réunies avec une solution aqueuse saturée en bicarbonate de sodium puis une solution aqueuse saturée en chlorure de sodium et on les sèche sur sulfate de sodium. Après évaporation du solvant, le résidu obtenu est purifié par chromatographie sur gel de silice en éluant avec un mélange 98/2 de dichlorométhane et de méthanol.
On obtient 2,33 g de produit sous forme de solide beige.
PF(°C) : 90-92°C 4.6. (+/-)-2-[6'-(4-Fluoro-phényl)-3,4,5,6-tétrahydro-2H-[1,2']bipyridinyl-4-yl]-éthylcarbamate de 2,2,2-trifluoro-1-thiazol-2-yléthyle
On chauffe à 80°C pendant 12 heures, une solution de 0,465 g (1 mmole) de 2-[6'-(4-fluoro-phényl)-3,4,5,6-tétrahydro-2H-[1,2']bipyridinyl-4-yl]-éthylcarbamate de 4-nitro-phényle, préparé à l'étape 4.4. , de 0,19 g (1,5 mmoles) de *N*,*N-*diisopropyléthylamine, de 0,006 g (0,05 mmole) de *N,N-*diméthylaminopyridine et de 0,20 g (1,1 mmole) de 2,2,2-trifluoro-1-thiazol-2-yl-éthanol, obtenu à l'étape 4.5., dans 5 ml de 1,2-dichloroéthane.
On laisse revenir à température ambiante, on sépare l'insoluble par filtration et on concentre le filtrat sous pression réduite. On reprend le résidu par du dichlorométhane et de l'eau, on sépare la phase aqueuse, on l'extrait deux fois avec du dichlorométhane, on lave successivement les phases organiques réunies avec une solution aqueuse de soude 1N puis une solution aqueuse saturée en chlorure de sodium et on les sèche sur sulfate de sodium. Après évaporation du solvant, on purifie le résidu obtenu par chromatographie sur gel de silice en éluant avec un mélange 98/2 de dichlorométhane et de méthanol.
On obtient ainsi 0,23 g de produit pur sous forme de poudre blanche.
LC-MS : M+H = 509
PF(°C) : 121-123°C
RMN ¹H (DMSO) δ (ppm) : 8,1 (m, 3H) ; 7,95 (m, 2H) ; 7,6 (t, 1H); 7,3 (t, 2H) ; 7,20 (d, 1H); 6,80 (d, 1H); 6,60 (m, 1H) ; 4,40 (large d, 2H) ; 3,1 (m, 2H) ; 2,8 (m, 2H); 1,75 (large d, 2H) ; 1,55 (m, 1H) ; 1,4 (m, 2H) ; 1,1 (m, 2H).

### Exemple 5 (Composé N°52)

### (+/-)-2-[6'-(4-Fluoro-phényl)-3,4,5,6-tétrahydro-2H-[1,2']bipyridinyl-4-yl]-éthylcarbamate de 2,2,2-trifluoro-1-thiazol-4-yléthyle

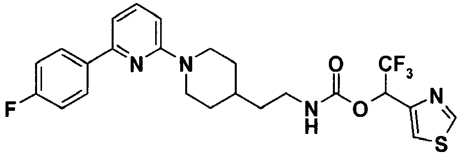

### 5.1. 2,2,2-Trifluoro-1-thiazol-4-yl-éthanol

On procède suivant la méthode décrite dans l'exemple 4 (étape 4.5.). A partir de 1 g (8,84 mmoles) de thiazole-4-carboxaldéhyde, de 0,10 ml (0,10 mmole) d'une solution de fluorure de tétrabutylammonium 1M dans le THF, de 1,38 g (9,72 mmoles) de trifluorométhyltriméthylsilane (TMS-CF₃), et après chromatographie sur gel de silice en éluant avec un mélange 98/2 de dichlorométhane et de méthanol, on obtient 0,54 g de produit pur sous forme d'huile incolore.

### 5.2. (+/-)-2-[6'-(4-Fluoro-phényl)-3,4,5,6-tétrahydro-2H-[1,2']bipyridinyl-4-yl]-éthylcarbamate de 2,2,2-trifluoro-1-thiazol-4-yléthyle

On procède suivant le mode opératoire décrit dans l'exemple 4 (étape 4.6.). A partir de 0,183 g (1 mmole) de 2-[6'-(4-fluoro-phényl)-3,4,5,6-tétrahydro-2H-[1,2']bipyridinyl-4-yl]-éthylcarbamate de 4-nitro-phényle, décrit dans l'exemple 4 (étape 4.4.), de 0,19 g (1,5 mmoles) de *N,N-*diisopropyléthylamine, de 0,006 g (0,05 mmole) de *N,N-*diméthylaminopyridine, de 0,51 g (1,1 mmoles) de 2,2,2-trifluoro-1-thiazol-4-yl-éthanol, obtenu à l'étape 5.1., et après chromatographie sur gel de silice en éluant avec un mélange 40/60 d'acétate d'éthyle et de cyclohexane, suivie d'une cristallisation dans un mélange de diéthyléther et d'hexane, on obtient 0,240 g de produit pur sous forme de poudre blanche.
LC-MS : M+H = 509
PF(°C) : 79-83°C
RMN ¹H (DMSO) δ (ppm) : 9,2 (s, 1H); 8,1 (dd, 2H) ; 8 (s, 1H) ; 7,85 (large t, 1H) ; 7,6 (dd, 1H) ; 7,35 (t, 2H) ; 7,15 (d, 1H); 6,80 (d, 1H) ; 6,45 (m, 1H) ; 4,40 (large d, 2H) ; 3,15 (m, 2H) ; 2,8 (m, 2H) ; 1,75 (large d, 2H) ; 1,55 (m, 1H) ; 1,4 (m, 2H) ; 1,1 (m, 2H).

### Exemple 6 (Composé N°55)

### (+/-)-2-[6'-(4-Fluoro-phényl)-3,4,5,6-tétrahydro-2H-[1,2']bipyridinyl-4-yl]-éthylcarbamte de 2,2,2-trifluoro-1-thiazol-5-yléthyle

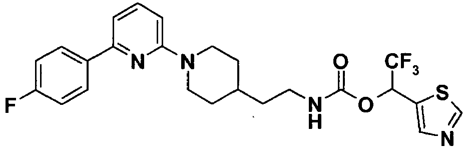

### 6.1. 2,2,2-Trifluoro-1-thiazol-5-yl-éthanol

On procède suivant la méthode décrite dans l'exemple 4 (étape 4.5.). A partir de 2 g (17,68 mmoles) de thiazole-5-carboxaldéhyde, de 0,88 ml (0,88 mmole) d'une solution de fluorure de tétrabutylammonium 1M dans le THF, de 2,765 g (19,44 mmoles) de trifluorométhyltriméthylsilane (TMS-CF₃), et après chromatographie sur gel de silice en éluant avec un mélange 98/2 de dichlorométhane et de méthanol, on obtient 2,23 g de produit pur sous forme d'huile incolore.

### 6.2. (+/-)-2-[6'-(4-Fluoro-phényl)-3,4,5,6-tétrahydro-2H-[1,2']bipyridinyl-4-yl]-éthylcarbamate de 2,2,2-trifluoro-1-thiazol-5-yléthyle

On procède suivant le mode opératoire décrit dans l'exemple 4 (étape 4.6.). A partir de 0,464 g (1 mmole) de 2-[6'-(4-fluoro-phényl)-3,4,5,6-tétrahydro-2H-[1,2']bipyridinyl-4-yl]-éthylcarbamate de 4-nitro-phényle, décrit dans l'exemple 4 (étape 4.4.), de 0,19 g (1,5 mmoles) de *N*,*N-*diisopropyléthylamine, de 0,006 g (0,05 mmole) de *N,N-*diméthylaminopyridine, de 0,201 g (1,1 mmoles) de 2,2,2-trifluoro-1-thiazol-5-yl-éthanol, obtenu à l'étape 6.1., et après chromatographie sur gel de silice en éluant avec un mélange 98/2 de dichlorométhane et de méthanol, on obtient 0,240 g de produit pur sous forme de gomme.
LC-MS : M+H = 509
RMN ¹H (DMSO) δ (ppm) : 9,3 (s, 1H) ; 8,2 (s, 1H) ; 8,1 (dd, 2H) ; 7,85 (large t, 1H); 7,6 (dd, 1H) ; 7,25 (dd, 2H) ; 7,15 (d, 1H) ; 6,90 (m, 1H); 6,80 (d, 1H) ; 4,40 (large d, 2H) ; 3,15 (m, 2H); 2,8 (m, 2H) ; 1,75 (large d, 2H) ; 1,55 (m, 1H) ; 1,4 (m, 2H); 1,1 (m, 2H).

### Exemple 7 (Composé N°106)

### (+/-)-2-[1-(6-Chloro-quinolin-2-yl)-pipéridin-4-yl]-éthylcarbamate de 2,2,2-trifluoro-1-thiazol-2-yléthyle

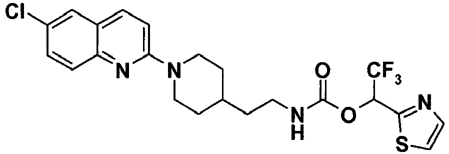

### 7.1. Méthanesulfonate de 2-[1-(6-chloro-quinolin-2-yl)-pipéridin-4-yl]-éthyle

A une solution de 4 g (13,76 mmoles) de 2-[1-(6-Chloroquinolin-2-yl)-pipéridin-4-yl]-éthanol (W02004/099176), de 3,55 g (27,51 mmoles) de diisopropyléthylamine et de 0,84 g (6,88 mmoles) de N,N-diméthylaminopyridine dans 30 ml de dichlorométhane, refroidie à environ 0°C, on ajoute goutte à goutte, sous atmosphère inerte, une solution de 2,36 g (20,63 mmoles) de chlorure de mésyle dans 3 ml de dichlorométhane. On poursuit l'agitation à 0°C pendant deux heures puis à température ambiante pendant une heure.
On ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite.
On obtient ainsi 5,1 g de produit sous forme d'huile utilisé tel quel dans l'étape suivante.

### 7.2. 2-[4-(2-Azido-éthyl)-pipéridin-1-yl]-6-chloro-quinoline

On porte au reflux pendant 4 heures, sous atmosphère inerte, une solution de 5 g (13,55 mmoles) de méthanesulfonate de 2-[1-(6-chloro-quinolin-2-yl)-pipéridin-4-yl]-éthyle, préparé à l'étape 7.1. et de 1,76 g (27,11 mmoles) d'azoture de sodium dans 30 ml de N,N-diméthylformamide.
On laisse revenir à température ambiante puis on concentre sous pression réduite. On reprend le résidu par du dichlorométhane et de l'eau, on sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation du solvant, on obtient 3,8 g de produit sous forme d'huile utilisé tel quel dans l'étape suivante.

### 7.3. 2-[1-(6-Chloro-quinolin-2-yl)-pipéridin-4-yl]-éthylamine

A une solution de 3,5 g (11,08 mmoles) de 2-[4-(2-azido-éthyl)-pipéridin-1-yl]-6-chloro-quinoline, obtenu à l'étape 7.2. dans 100 ml de THF/Eau (1/1), on ajoute par petite portion, à température ambiante, 4,36 g (16,62 mmoles) de triphénylphosphine. On poursuit l'agitation à température ambiante pendant dix heures.
On concentre sous pression réduite. On ajoute de l'acétate d'éthyle, on sépare la phase aqueuse, on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. Après chromatographie sur gel de silice en éluant avec un mélange 90/10/1 de dichlorométhane, de méthanol et d'ammoniaque à 28%, on obtient 1,77 g de produit pur sous forme d'huile qui cristallise à température ambiante.
PF (°C) : 68-70°C
RMN ¹H (CDCl₃) δ (ppm) : 7,7 (d, 1H) ; 7,5 (m, 2H) ; 7,35 (m, 1H) ; 6,95 (d, 1H) ; 4,45 (large d, 2H) ; 2,9 (large td, 2H) ; 2,7 (t, 2H) ; 1,7 (m, 2H) ; 1,6-1,1 (m, 5H).

### 7.4. 2-[1-(6-Chloro-quinolin-2-yl)-pipéridin-4-yl]-éthyl}-carbamate de 4-nitro-phényle

On procède suivant la méthode décrite dans l'exemple 4 (étape 4.4.). A partir de 5 g (17,25 mmoles) de 2-[1-(6-chloroquinolin-2-yl)-pipéridin-4-yl]-éthylamine, préparé à l'étape 7.3., de 3,825 g (18,98 mmoles) de chloroformiate de 4-nitrophényle, de 4,46 g (34,51 mmoles) de N,N-diisopropyléthylamine, de 0,105 g (0,86 mmole) de N,N-diméthylaminopyridine, et après trituration dans un mélange de diisopropyléther et d'hexane , on obtient 7,8 g de produit pur sous forme de poudre blanche.
PF(°C) : 80-84°C

### 7.5. (+/-)-2-[1-(6-Chloro-quinolin-2-yl)-pipéridin-4-yl]-éthylcarbamate de 2,2,2-trifluoro-1-thiazol-2-yléthyle

On procède suivant le mode opératoire décrit dans l'exemple 4 (étape 4.6.). A partir de 0,455 g (1 mmole) de 2-[1-(6-chloroquinolin-2-yl)-pipéridin-4-yl]-éthyl}-carbamate de 4-nitrophényle, obtenu à l'étape 7.4., de 0,19 g (1,5 mmoles) de N,N-diisopropyléthylamine, de 0,006 g (0,05 mmole) de N,N-diméthylaminopyridine, de 0,201 g (1,1 mmoles) de 2,2,2-trifluoro-1-thiazol-2-yl-éthanol, décrit dans l'exemple 5 (étape 5.5.), et après chromatographie sur gel de silice en éluant avec un mélange 98/2 de dichlorométhane et de méthanol, suivie d'une cristallisation dans un mélange de diéthyléther et d'hexane, on obtient 0,3 g de produit pur sous forme de poudre blanche.
LC-MS : M+H = 499
PF(°C) : 114-116°C
RMN ¹H (DMSO) δ (ppm) : 8,1 (t, 1H) ; 8 (m, 2H) ; 7,8 (s, 1H); 7,5 (m, 3H); 7,30 (d, 1H) ; 6,60 (m, 1H) ; 4,55 (large d, 2H) ; 3,15 (m, 2H) ; 2,9 (m, 2H) ; 1,8 (large d, 2H) ; 1,55 (m, 1H) ; 1,4 (m, 2H) ; 1,1 (m, 2H).

### Exemple 8 (Composé N°38)

### 2-[5'-(4-Ethoxy-phényl)-3,4,5,6-tétrahydro-2H-[1,2']bipyridinyl-4-yl]-éthylcarbamate de thiazol-2-ylméthyle

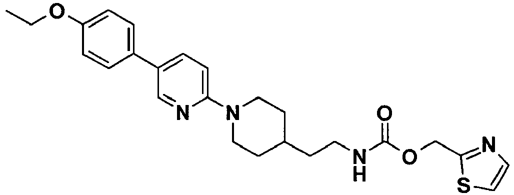

### 8.1. 2-(5'-Bromo-3,4,5,6-tétrahydro-2H-[1,2']bipyridinyl-4-yl)-éthylcarbamate de tert-butyle

Dans un autoclave, on introduit 10,37 g (43,80 mmoles) de 2,5-dibromopyridine, 10 g (43,80 mmoles) de 2-pipéridin-4-yl-éthylcarbamate de tert-butyle et 6,05 g (43,8 mmoles) de carbonate de potassium. On chauffe ensuite à 130°C pendant 12 heures.
On laisse revenir à température ambiante puis on reprend le mélange réactionnel par du chloroforme et une solution aqueuse saturée en hydrogénocarbonate de sodium. On sépare la phase aqueuse, on l'extrait deux fois avec du chloroforme, on lave les phases organiques réunies avec une solution aqueuse saturée en chlorure de sodium, on les sèche sur sulfate de sodium et on concentre le filtrat sous pression réduite. Après chromatographie sur gel de silice en éluant avec un mélange 95/5 de dichlorométhane et de méthanol, on obtient 6,9 g de produit pur sous forme de poudre blanche.
PF(°C) : 108-110°C

### 8.2. 2-(5'-Bromo-3,4,5,6-tétrahydro-2H-[1,2']bipyridinyl-4-yl)-éthylamine

A une solution de 6,9 g (17,95 mmoles) de 2-(5'-bromo-3,4,5,6-tétrahydro-2H-[1,2']bipyridinyl-4-yl)-éthylcarbamate de tert-butyle, obtenu à l'étape 8.1., dans 100 ml de dichlorométhane, refroidie par un bain de glace/eau, on ajoute lentement 20,47 g (179,54 mmoles) d'acide trifluoroacétique. On poursuit l'agitation à température ambiante pendant 2 heures. On verse le mélange réactionnel dans un mélange d'eau glacée et d'ammoniaque à 28 %. On décante, on extrait deux fois la phase aqueuse par du dichlorométhane, on lave les phases organiques réunies avec une solution aqueuse saturée en chlorure de sodium, on les sèche sur sulfate de sodium et on concentre sous pression réduite.
On obtient 4,9 g de produit sous forme d'huile utilisé tel quel dans l'étape suivante.

### 8.3. 2-(5'-Bromo-3,4,5,6-tétrahydro-2H-[1,2']bipyridinyl-4-yl)-éthylcarbamate de thiazol-2-ylméthyle

On procède suivant le mode opératoire décrit dans l'exemple 1 (étape 1.4.). A partir de 4,3 g (15,13 mmoles) de 2-(5'-bromo-3,4,5,6-tétrahydro-2H-[1,2']bipyridinyl-4-yl)-éthylamine, obtenu à l'étape 8.2., de 4,66 g (16,64 mmoles) de (4-nitrophényl)-carbonate de thiazol-2-ylméthyle (EP486948A2), de 2,93 g (22,70 mmoles) de N,N-diisopropyléthylamine, de 0,09 g (0,76 mmole) de N,N-diméthylaminopyridine, et après chromatographie sur gel de silice en éluant avec un mélange 20/80 d'acétate d'éthyle et de cyclohexane, on obtient 2,6 g de produit pur sous forme de poudre blanche.

### 8.4. 2-[5'-(4-Ethoxy-phényl)-3,4,5,6-tétrahydro-2H-[1,2']bipyridinyl-4-yl]-éthylcarbamate de thiazol-2-ylméthyle

Sous atmosphère inerte, on introduit 0,425 g (1 mmole) de 2-(5'-bromo-3,4,5,6-tétrahydro-2H-[1,2']bipyridinyl-4-yl)-éthylcarbamate de thiazol-2-ylméthyle, obtenu à l'étape 8.3., 0,2 g (1,2 mmoles) d'acide 4-éthoxyphénylboronique, 0,977 g (3 mmoles) de carbonate de cesium en suspension dans 5 ml d'un mélange 9/1 de tétrahydrofurane et d'eau. On ajoute ensuite 0,082 g (0,1 mole) de PdCl₂dppf.CH₂Cl₂. On chauffe ensuite à environ 75°C pendant 12 heures.
On laisse revenir à température ambiante, on sépare les sels par filtration sur célite, puis on reprend le filtrat avec de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 98/2 de dichlorométhane et de méthanol. Le solide obtenu est ensuite recristallisé dans l'isopropanol.
On obtient 0,39 g de produit sous forme de poudre blanche.
LC-MS : M+H = 467
PF(°C) : 157-159°C
RMN ¹H (DMSO) δ (ppm) : 8,40 (s, 1H) ; 7,85 (d, 1H) ; 7,75 (m, 2H) ; 7,55 (d, 2H) ; 7,45 (t, 1H); 7 (d, 2H) ; 6,9 (d, 1H) ; 5,30 (s, 2H); 4,30 (large d, 2H); 4,1 (q, 2H) ; 3,1 (m, 2H) ; 2,8 (t, 2H); 1,8 (d, 2H) ; 1,7 (m, 1H) ; 1,4 (m, 2H) ; 1,3 (t, 3H) ; 1,1 (m, 2H).

### Exemple 9 (Composé N°117)

### 2-[1-(6-Fluoro-quinolin-2-yl)-pipéridin-4-yl]-éthylcarbamate de thiazol-5-ylméthyle

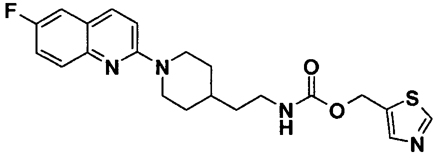

### 9.1. 4-[2-(4-Nitro-phénoxycarbonylamino)-éthyl]-pipéridine-1-carboxylate de tert-butyle

On procède suivant le mode opératoire décrit dans l'exemple 1 (étape 1.4.). A partir de 5 g (21,90 mmoles) de 4-(2-amino-éthyl)-pipéridine-1-carboxylate de tert-butyle, de 4,63 g (23 mmoles) de chloroformiate de 4-nitro-phényle, de 5,66 g (43,80 mmoles) de diisopropyléthylamine et de 0,134 g (1,09 mmoles) de N,N-diméthylaminopyridine, on obtient 8,6 g de produit sous forme d'huile utilisé tel quel dans l'étape suivante.

### 9.2. 4-[2-(Thiazol-5-ylméthoxycarbonylamino)-éthyl]-pipéridine-1-carboxylate de tert-butyle

On procède suivant le mode opératoire décrit dans l'exemple 4 (étape 4.6.). A partir de 8,6 g (21, 86 mmoles) de 4-[2-(4-nitro-phénoxycarbonylamino)-éthyl]-pipéridine-1-carboxylate de tert-butyle , obtenu à l'étape 9.1., de 2,77 g (24,04 mmoles) de thiazol-2-yl-méthanol, de 5,65 g (43,72 mmoles) de N,N-diisopropyléthylamine et de 0,134 g (1,09 mmole) de N,N-diméthylaminopyridine, on obtient 3,6 g de produit sous forme d'huile utilisé tel quel dans l'étape suivante.

### 9.3. Chlorhydrate de 2-pipéridin-4-yl-éthylcarbamate de thiazol-5-ylméthyle

A une solution de 3,6 g (9,74 mmoles) de 4-[2-(thiazol-5-ylméthoxycarbonylamino)-éthyl]-pipéridine-1-carboxylate de tert-butyle, obtenu à l'étape 9.2., dans 97 ml de diéthyléther, refroidie par un bain de glace/eau, on ajoute lentement 40 ml (160 mmoles) d'une solution d'acide chlorhydrique 4N dans le dioxane. On poursuit l'agitation à température ambiante pendant 12 heures.
Après évaporation sous pression réduite, on obtient 2,4 g de produit sous forme de chlorhydrate utilisé tel quel dans l'étape suivante.

### 9.4. 2-[1-(6-Fluoro-quinolin-2-yl)-pipéridin-4-yl]-éthylcarbamate de thiazol-5-ylméthyle

Dans un tube scellé, on introduit 0,09 g (0,39 mmole) de 2-bromo-6-fluoro-quinoline, 0,1 g (0,33 mmoles) de chlorhydrate de 2-pipéridin-4-yl-éthylcarbamate de thiazol-5-ylméthyle, obtenu à l'étape 9.3. et 0,2 ml (1,14 mmoles) de N,N-diisopropyléthylamine. On chauffe ensuite à 100°C pendant 12 heures.
On laisse revenir à température ambiante puis on reprend le mélange réactionnel par du dichlorométhane et une solution aqueuse saturée en chlorure d'ammonium. On sépare la phase aqueuse, on l'extrait deux fois avec du dichlorométhane, on lave les phases organiques réunies avec une solution aqueuse saturée en chlorure de sodium, on les sèche sur sulfate de sodium et on concentre le filtrat sous pression réduite. Après chromatographie sur gel de silice en éluant avec un mélange 95/5 de dichlorométhane et de méthanol, on obtient 0,039 g de produit pur sous forme de poudre blanche.
LC-MS : M+H = 415
PF(°C) : 100-102°C
RMN ¹H (DMSO) δ (ppm) : 9,1 (s, 1H) ; 8 (d, 1H); 7,9 (s, 1H); 7,7 (m, 1H) ; 7,60 (dd, 1H) ; 7,5 (m, 1H) ; 7,30 (m, 2H) ; 5,3 (s, 2H) ; 4,50 (large d, 2H) ; 3,10 (m, 2H) ; 2,85 (t, 2H) ; 1,9 (large d, 2H) ; 1,60 (m, 1H) ; 1,4 (m, 2H) ; 1,1 (m, 2H).

Le tableau 1 qui suit illustre les structures chimiques et les propriétés physiques de quelques composés selon l'invention. Dans ce tableau :
- dans la colonne « base ou sel », « base » représente un composé sous forme de base libre, « CF₃COOH » représente un composé sous forme de trifluoroacétate, « HCl » représente un composé sous forme de chlorhydrate ;
- dans la colonne « A », « 1 » correspond à un -CH₂-, « 2 » correspond à un -CH₂-CH₂- ;
- tous les composés comportant un carbone asymétrique sont sous forme de mélange racémique.

**Tableau 1**

| | | | | | | | | | |
|---|---|---|---|---|---|---|---|---|---|
| 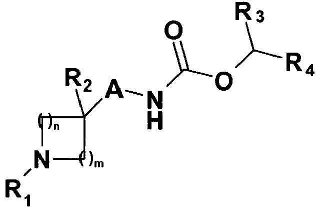 | | | | | | | | | |
| N° | R₁ | m | n | A | R₂ | R₃ | R₄ | base ou sel | PF (°C) ou M+H |
| 1. | 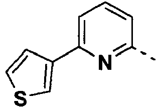 | 2 | 2 | 1 | H | H | thiazol-4yl | CF₃COOH | 415 |
| 2. | | 2 | 2 | 2 | H | H | thiazol-4yl | CF₃COOH | 429 |
| 3. | 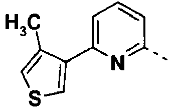 | 2 | 2 | 1 | H | H | thiazol-4yl | CF₃COOH | 429 |
| 4. | | 2 | 2 | 2 | H | H | thiazol-4yl | CF₃COOH | 443 |
| 5. | 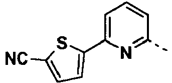 | 2 | 2 | 2 | H | H | thiazol-4yl | CF₃COOH | 454 |
| 6. | 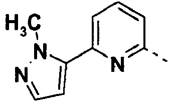 | 2 | 2 | 2 | H | H | thiazol-2yl | HCl | 125-127°C |
| 7. | | 2 | 2 | 1 | H | H | thiazol-4yl | CF₃COOH | 413 |
| 8. | | 2 | 2 | 2 | H | H | thiazol-4yl | base | 94-95°C |

| N° | R₁ | m | n | A | R₂ | R₃ | R₄ | base ou sel | PF (°C) ou M+H |
|---|---|---|---|---|---|---|---|---|---|
| 9. | | 2 | 2 | 2 | H | H | thiazol-2yl | base | 90°C (déc.) |
| 10. | 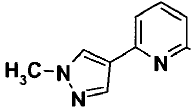 | 2 | 2 | 2 | H | H | thiazol-4yl | base | 117-119°C |
| 11. | 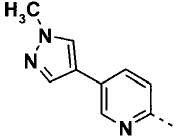 | 2 | 2 | 2 | H | H | thiazol-4yl | base | 153-155°C |
| 12. | 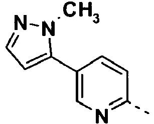 | 2 | 2 | 2 | H | H | thiazol-4yl | base | 107-109°C |
| 13. | | 2 | 2 | 2 | H | H | thiazol-2yl | base | 132-134°C |
| 14. | 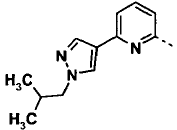 | 2 | 2 | 1 | H | H | thiazol-4yl | CF₃COOH | 455 |
| 15. | | 2 | 2 | 2 | H | H | thiazol-4yl | CF₃COOH | 469 |
| 16. | 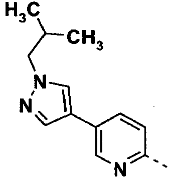 | 2 | 2 | 2 | H | H | thiazol-4yl | base | 132-134°C |
| 17. | | 2 | 2 | 1 | H | H | thiazol-4yl | CF₃COOH | 428 |
| 18. | 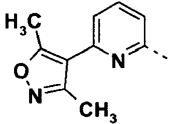 | 2 | 2 | 2 | H | H | thiazol-4yl | CF₃COOH | 442 |
| 19. | | 2 | 2 | 1 | H | H | thiazol-4yl | CF₃COOH | 413 |
| 20. | 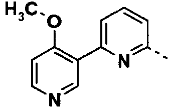 | 2 | 2 | 2 | H | H | thiazol-4yl | CF₃COOH | 454 |
| 21. | | 2 | 2 | 2 | H | H | thiazol-2yl | base | 124-126°C |
| 22. | 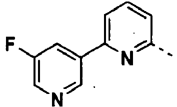 | 2 | 2 | 2 | H | H | thiazol-4yl | base | 123-125°C |
| 23. | 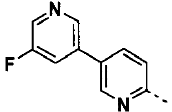 | 2 | 2 | 2 | H | H | thiazol-4yl | base | 133-135°C |

| N° | R₁ | m | n | A | R₂ | R₃ | R₄ | base ou sel | PF (°C) où M+H |
|---|---|---|---|---|---|---|---|---|---|
| 24. | 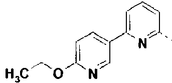 | 2 | 2 | 2 | H | H | thiazol-4yl | CF₃COOH | 468 |
| 25. | 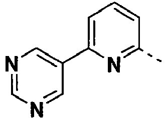 | 2 | 2 | 1 | H | H | thiazol-4yl | CF₃COOH | 411 |
| 26. | 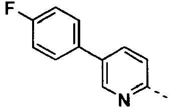 | 2 | 1 | 1 | H | H | thiazol-2yl | base | 96-98°C |
| 27. | | 2 | 1 | 1 | H | H | thiazol-4yl | base | 106-108°C |
| 28. | | 2 | 2 | 2 | H | H | thiazol-2yl | base | 131-133°C |
| 29. | | 2 | 2 | 2 | H | CF₃ | thiazol-2yl | base | 132-134°C |
| 30. | | 2 | 2 | 2 | H | H | thiazol-4yl | base | 130-132°C |
| 31. | | 2 | 2 | 2 | H | CF₃ | thiazol-4yl | base | 120-122°C |
| 32. | | 2 | 2 | 2 | H | H | 2-CH₃-thiazol-4yl | base | 149-151°C |
| 33. | | 2 | 2 | 2 | H | H | thiazol-5yl | base | 100-102°C |
| 34. | | 2 | 2 | 2 | H | CF₃ | thiazol-5yl | base | 165-167°C |
| 35. | 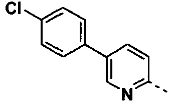 | 2 | 2 | 2 | H | H | thiazol-2yl | base | 145-149°C |
| 36. | 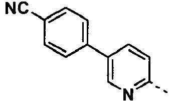 | 2 | 2 | 2 | H | H | thiazol-2yl | base | 189-191°C |
| 37. | | 2 | 2 | 2 | H | H | thiazol-5yl | base | 147-149°C |
| 38. | 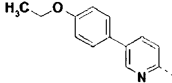 | 2 | 2 | 2 | H | H | thiazol-2yl | base | 157-159°C |
| 39. | | 2 | 2 | 2 | H | H | thiazol-4yl | base | 160-162°C |
| 40. | | 2 | 2 | 2 | H | H | thiazol-5yl | base | 170-172°C |
| 41. | 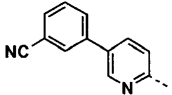 | 2 | 2 | 2 | H | H | thiazol-2yl | base | 114-116°C |
| 42. | 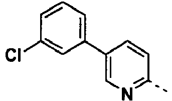 | 2 | 2 | 2 | H | H | thiazol-2yl | base | 101-103°C |
| 43. | 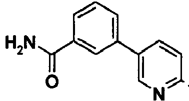 | 2 | 2 | 2 | H | H | thiazol-4yl | base | 195-197°C |
| .44. | 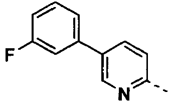 | 2 | 2 | 2 | H | H | thiazol-2yl | base | 101-103°C |
| N° | R₁ | m | n | A | R₂ | R₃ | R₄ | base ou sel | PF (°C) ou M+H |
| 45. | | 2 | 2 | 1 | H | H | thiazol-4yl | CF₃COOH | 477 |
| 46. | 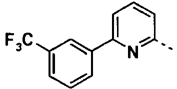 | 2 | 2 | 2 | H | H | thiazol-4yl | CF₃COOH | 491 |
| 47. | 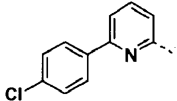 | 2 | 2 | 1 | H | H | thiazol-4yl | CF₃COOH | 443 |
| 48. | | 2 | 2 | 2 | H | H | thiazol-4yl | CF₃COOH | 457 |
| 49. | 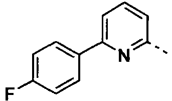 | 2 | 2 | 2 | H | H | thiazol-2yl | base | 95-97 °C |
| 50. | | 2 | 2 | 2 | H | CF₃ | thiazol-2yl | base | 121-123°C |
| 51. | | 2 | 2 | 2 | H | H | thiazol-4yl | base | 118-120°C |
| 52. | | 2 | 2 | 2 | H | CF₃ | thiazol-4yl | base | 79-83°C |
| 53. | | 2 | 2 | 2 | H | H | 2-CH₃-thiazol-4yl | base | 127-129°C |
| 54. | | 2 | 2 | 2 | H | H | thiazol-5yl | base | 78-82°C |
| 55. | | 2 | 2 | 2 | H | CF₃ | thiazol-5yl | base | 509 |
| 56. | 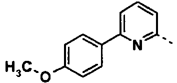 | 2 | 2 | 1 | H | H | thiazol-4yl | CF₃COOH | 439 |
| 57. | | 2 | 2 | 2 | H | H | thiazol-4yl | CF₃COOH | 453 |
| 58. | 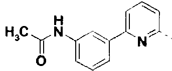 | 2 | 2 | 1 | H | H | thiazol-4yl | CF₃COOH | 466 |
| 59. | | 2 | 2 | 2 | H | H | thiazol-4yl | CF₃COOH | 480 |
| 60. | 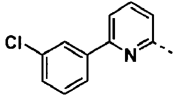 | 2 | 2 | 1 | H | H | thiazol-4yl | CF₃COOH | 443 |
| 61. | | 2 | 2 | 2 | H | H | thiazol-4yl | CF₃COOH | 457 |
| 62. | 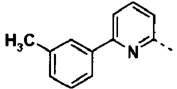 | 2 | 2 | 1 | H | H | thiazol-4yl | CF₃COOH | 423 |
| 63. | 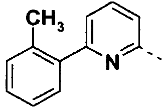 | 2 | 2 | 2 | H | H | thiazol-4yl | CF₃COOH | 437 |
| 64. | 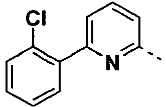 | 2 | 2 | 1 | H | H | thiazol-4yl | CF₃COOH | 443 |
| 65. | 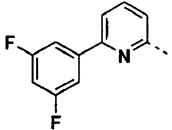 | 2 | 2 | 1 | H | H | thiazol-4yl | CF₃COOH | 445 |
| 66. | | 2 | 2 | 2 | H | H | thiazol-4yl | CF₃COOH | 459 |
| 67. | 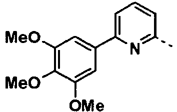 | 2 | 2 | 1 | H | H | thiazol-4yl | CF₃COOH | 499 |

| N° | R₁ | m | n | A | R₂ | R₃ | R₄ | base ou sel | PF (°C) ou M+H |
|---|---|---|---|---|---|---|---|---|---|
| 68. | 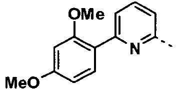 | 2 | 2 | 2 | H | H | thiazol-4yl | CF₃COOH | 483 |
| 69. | 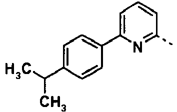 | 2 | 2 | 1 | H | H | thiazol-4yl | CF₃COOH | 451 |
| 70. | | 2 | 2 | 2 | H | H | thiazol-4yl | CF₃COOH | 465 |
| 71. | 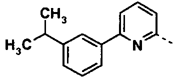 | 2 | 2 | 1 | H | H | thiazol-4yl | CF₃COOH | 451 |
| 72. | | 2 | 2 | 2 | H | H | thiazol-4yl | CF₃COOH | 465 |
| 73. | 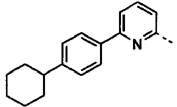 | 2 | 2 | 1 | H | H | thiazol-4yl | CF₃COOH | 491 |
| 74. | | 2 | 2 | 2 | H | H | thiazol-4yl | CF₃COOH | 505 |
| 75. | 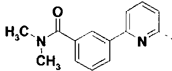 | 2 | 2 | 1 | H | H | thiazol-4yl | CF₃COOH | 480 |
| 76. | | 2 | 2 | 2 | H | H | thiazol-4yl | CF₃COOH | 494 |
| 77. | 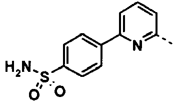 | 2 | 2 | 2 | H | H | thiazol-4yl | CF₃COOH | 502 |
| 78. | 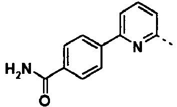 | 2 | 2 | 1 | H | H | thiazol-4yl | CF₃COOH | 452 |
| 79. | | 2 | 2 | 2 | H | H | thiazol-4yl | CF₃COOH | 466 |
| 80. | 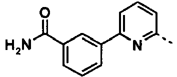 | 2 | 2 | 1 | H | H | thiazol-4yl | CF₃COOH | 452 |
| 81. | | 2 | 2 | 2 | H | H | thiazol-4yl | CF₃COOH | 466 |
| 82. | 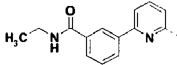 | 2 | 2 | 1 | H | H | thiazol-4yl | CF₃COOH | 480 |
| 83. | | 2 | 2 | 2 | H | H | thiazol-4yl | CF₃COOH | 494 |
| 84. | 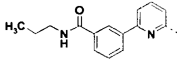 | 2 | 2 | 1 | H | H | thiazol-4yl | CF₃COOH | 494 |
| 85. | 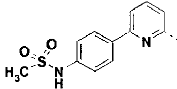 | 2 | 2 | 2 | H | H | thiazol-4yl | CF₃COOH | 516 |
| 86. | 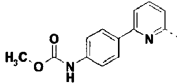 | 2 | 2 | 1 | H | H | thiazol-4yl | CF₃COOH | 482 |
| 87. | | 2 | 2 | 2 | H | H | thiazol-4yl | CF₃COOH | 496 |
| 88. | 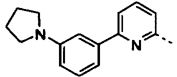 | 2 | 2 | 2 | H | H | thiazol-4yl | CF₃COOH | 492 |
| 89. | 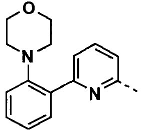 | 2 | 2 | 1 | H | H | thiazol-4yl | CF₃COOH | 494 |
| 90. | | 2 | 2 | 2 | H | H | thiazol-4yl | CF₃COOH | 508 |
| 91. | 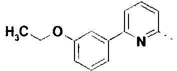 | 2 | 2 | 2 | H | H | thiazol-4yl | CF₃COOH | 467 |
| 92. | 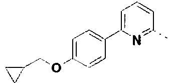 | 2 | 2 | 2 | H | H | thiazol-4yl | CF₃COOH | 493 |
| 93. | 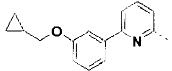 | 2 | 2 | 1 | H | H | thiazol-4yl | CF₃COOH | 479 |
| 94. | | 2 | 2 | 2 | H | H | thiazol-4yl | CF₃COOH | 493 |
| 95. | 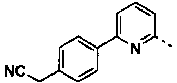 | 2 | 2 | 1 | H | H | thiazol-4yl | CF₃COOH | 448 |
| 96. | 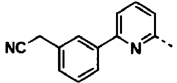 | 2 | 2 | 1 | H | H | thiazol-4yl | CF₃COOH | 448 |
| 97. | 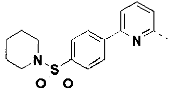 | 2 | 2 | 1 | H | H | thiazol-4yl | CF₃COOH | 556 |
| 98. | | 2 | 2 | 2 | H | H | thiazol-4yl | CF₃COOH | 570 |
| 99. | 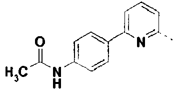 | 2 | 2 | 1 | H | H | thiazol-4yl | CF₃COOH | 466 |
| 100 | | 2 | 2 | 2 | H | H | thiazol-4yl | CF₃COOH | 479 |
| 101 | 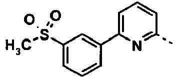 | 2 | 2 | 2 | H | H | thiazol-4yl | CF₃COOH | 501 |
| 102 | 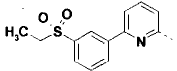 | 2 | 2 | 1 | H | H | thiazol-4yl | CF₃COOH | 501 |
| 103 | | 2 | 2 | 2 | H | H | thiazol-4yl | CF₃COOH | 515 |
| 104 | 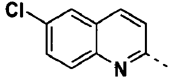 | 2 | 2 | 2 | H | H | thiazol-2yl | base | 121-123°C |
| 105 | | 2 | 2 | 2 | H | H | 4-CF₃-thiazol-2yl | base | 128-129°C |
| 106 | | 2 | 2 | 2 | H | CF₃ | thiazol-2yl | base | 114-116°C |
| 107 | | 2 | 2 | 2 | H | CH₃ | thiazol-4yl | base | 142-144°C |
| 108 | | 2 | 2 | 2 | H | CF₃ | thiazol-4yl | base | 111-113°C |
| 109 | | 2 | 2 | 2 | H | H | thiazol-4yl | base | 126-128°C |
| 110 | | 2 | 2 | 2 | H | H | 2-CH₃-thiazol-4yl | base | 142-144°C |
| 111 | | 2 | 2 | 2 | H | H | 2-CF₃-thiazol-4yl | base | 148-150°C |
| 112 | | 2 | 2 | 2 | H | H | 2-Cl-thiazol-4yl | base | 130-132°C |
| 113 | | 2 | 2 | 2 | H | H | 2-(pyridin-3yl)-thiazol-4yl | base | 152-154°C |
| 114 | | 2 | 2 | 2 | H | H | 2-(pyridin-4yl)-thiazol-5yl | base | 155-157°C |
| 115 | | 2 | 2 | 2 | H | H | thiazol-5yl | base | 132-134°C |
| 116 | | 2 | 2 | 2 | H | CF₃ | thiazol-5yl | base | 131-133°C |
| 117 | 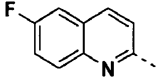 | 2 | 2 | 2 | H | H | thiazol-5yl | base | 100-102°C |
| 118 | 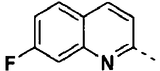 | 2 | 2 | 2 | H | H | thiazol-5yl | base | 115-117°C |
| 119 | 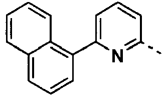 | 2 | 2 | 1 | H | H | thiazol-4yl | CF₃COOH | 459 |
| 120 | | 2 | 2 | 2 | H | H | thiazol-4yl | CF₃COOH | 473 |
| 121 | 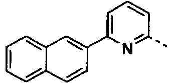 | 2 | 2 | 1 | H | H | thiazol-4yl | CF₃COOH | 459 |
| 122 | | 2 | 2 | 2 | H | H | thiazol-4yl | CF₃COOH | 473 |
| 123 | 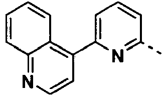 | 2 | 2 | 1 | H | H | thiazol-4yl | CF₃COOH | 460 |
| 124 | | 2 | 2 | 2 | H | H | thiazol-4yl | CF₃COOH | 474 |
| 125 | 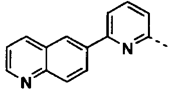 | 2 | 2 | 1 | H | H | thiazol-4yl | CF₃COOH | 460 |
| 126 | | 2 | 2 | 2 | H | H | thiazol-4yl | CF₃COOH | 474 |
| 127 | 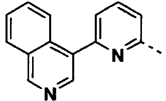 | 2 | 2 | 1 | H | H | thiazol-4yl | CF₃COOH | 460 |
| 128 | | 2 | 2 | 2 | H | H | thiazol-4yl | CF₃COOH | 474 |
| 129 | 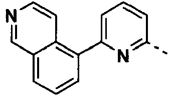 | 2 | 2 | 1 | H | H | thiazol-4yl | CF₃COOH | 460 |
| 130 | | 2 | 2 | 2 | H | H | thiazol-4yl | CF₃COOH | 474 |
| 131 | 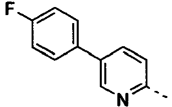 | 2 | 2 | 2 | H | H | 2-CONH₂-thiazol-4yl | base | 230-232°C |
| 132 | | 2 | 2 | 2 | H | H | 2-CONHCH₃-thiazol-4yl | base | 184-186°C |
| 133 | | 2 | 2 | 2 | H | H | 4-CONH₂-thiazol-2yl | base | 199-201°C |
| 134 | | 2 | 2 | 2 | H | H | 4-CONHCH₃-thiazol-2yl | base | 177-178°C |

| | | | | | | | | | |
|---|---|---|---|---|---|---|---|---|---|
| Méthodes LC-MS (M+H) : Acétonitrile + 0,5% acide trifluroacétique / H₂O + 0,05% acide trifluroacétique Colonnes : Waters Xbridge C18 4 ou YMC Jsphere 33*2 Débit : 1ml/min | | | | | | | | | |

Les composés de l'invention ont fait l'objet d'essais pharmacologiques permettant de déterminer leur effet inhibiteur de l'enzyme FAAH (Fatty Acid Amido Hydrolase).

L'activité inhibitrice a été mise en évidence dans un test radioenzymatique basé sur la mesure du produit d'hydrolyse de l'anandamide [éthanolamine 1-³H] par la FAAH *(*Life Sciences (1995), 56, 1999-2005 et Journal of Biochemical and Biophysical Methods (2004), 60(2), 171-177). Ainsi, les cerveaux de souris (moins le cervelet) sont prélevés et conservés à -80°C. Les homogénats membranaires sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/ml) est utilisé pour la réaction enzymatique, la dilution des composés et de l'anandamide [éthanolamine 1-³H]. Sont ajoutés successivement dans les puits, du tampon de réaction contenant la BSA (43µl/puit), les composés dilués testés à différentes concentrations (7µl/puit contenant 1% de DMSO) et la préparation membranaire (10µl/puit soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puit, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puit). Le mélange est agité 10 minutes puis la phase aqueuse contenant l'éthanolamine [1-³H] est récupérée par filtration sous-vide et comptée par scintillation liquide.
Dans ces conditions, les composés les plus actifs de l'invention présentent des CI₅₀ (concentration inhibant de 50 % l'activité enzymatique contrôle de la FAAH) comprises entre 0,001 et 1 µM, par exemple les composés n°28 et 30 ont des CI₅₀ respectives de 0,003 et 0,007 µM.
Il apparaît donc que les composés selon l'invention ont une activité inhibitrice sur l'enzyme FAAH.

L'activité *in vivo* des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 35 à 80 % le nombre d'étirements induits par le PBQ, dans une gamme de doses comprise entre 1 et 30 mg/kg. Par exemple, les composés n° 28 et n° 30 du tableau réduisent, respectivement, de 33% et de 80% le nombre d'étirements induits par le PBQ, à la dose de 30 mg/kg p.o. à 120 minutes.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes : la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures, les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.
Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) 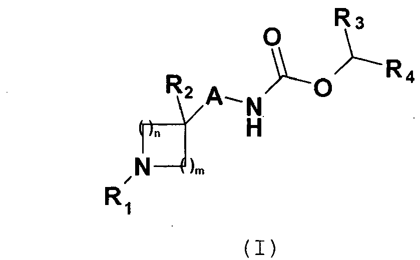 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
n représente un nombre entier égal à 1, 2 ou 3 et m représente un nombre entier égal à 1 ou 2;
A représente une liaison covalente ou un groupe C₁₋₈₋alkylène ;
Rᵢ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉,, NR₈SO₂R₉, NR₈SO₂NR₈R₉, COR₈, CO₂R₈, CONR₈R₉, SO₂R₈, SO₂NR₈R₉ ou -O-(C₁₋₃-alkylène) -O- ;
R₇ représente un groupe choisi parmi un furanyle, pyrrolyle, thiènyle, oxazolyle, isoxazolyle,- thiazolyle, isothiazolyle, imidazolyle, pyrazolyle,oxadiazole, thiadiazole, phényle, pyridinyle, pyridazinyle, pyrimidinyle, pyrazinyle, triazine, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un thiazole éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉, -O-(C₁₋₃-alkylène)-O-, phényle, phényloxy, benzyloxy, pyridinyle, pyrazinyle, pyridazinyle, triazinyle ou pyrimidinyle; les groupes phényle, phényloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène ;
R₈ et R₉ représentent indépendamment l'un de l'autre un atome d'hydrogène ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₆COR₉, un cycle lactame ; dans le cas de NR₈CO₂R₉, un cycle oxazolidinone, oxazinone ou oxazépinone;
dans le cas de NR₈SO₂R₉, un cycle sultame ; dans le cas de NR₈SO₂NR₈R₉, un cycle dioxyde de thiazolidine ou dioxyde de thiadiazinane ;
à l'état de base ou de sel d'addition à un acide ;
les composés suivants étant exclus :
- 2-(3-{[2-(4-chlorophenyl)-4-methylthiazol-5-yl]methoxycarbonylamino}piperidin-1-yl)benzoate de méthyle ;
- acide 2-(3-{[2-(4-chlorophenyl)-4-methylthiazol-5-yl]methoxycarbonylamino}piperidin-1-yl)benzoïque;
- 3-(3-{[2-(4-chlorophenyl)-4-methylthiazol-5-yl]methoxycarbonylamino}piperidin-1-yl)benzoate de méthyle ;
- acide 3-(3-{[2-(4-chlorophenyl)-4-methylthiazol-5-yl]methoxycarbonylamino}piperidin-1-yl)benzoïque.

2. Composé de formule (I) selon la revendication 1 **caractérisé en ce que** R₂ représente un atome d'hydrogène ; à l'état de base ou de sel d'addition à un acide.

3. Composé de formule (I) selon la revendication 1 ou 2 **caractérisé en ce que** n représente un nombre entier égal à 1 ou 2 et m représente un nombre entier égal à 2 ; à l'état de base ou de sel d'addition à un acide.

4. Composé de formule (I) selon l'une quelconque des revendications 1 à 3 **caractérisé en ce que** A représente un groupe C₁₋₈₋alkylène ; à l'état de base ou de sel d'addition à un acide.

5. Composé de formule (I) selon l'une quelconque des revendications 1 à 4 **caractérisé en ce que** R₁ représente un groupe R₅ éventuellement substitué par un ou plusieurs groupes R₆ et/ou R₇ ;
R₅ représente un groupe pyridinyle ou quinolinyle ;
R₆ représente un atome d'halogène, un groupe cyano, -CH₂CN, C₁₋₆-alkyle, C₁₋₆-alcoxy, C₁₋₆-haloalkyle, C₃₋₇-cycloalkyle, C₃₋₇-cycloalkyle-C₁₋₃-alkylène-O-, NR₈R₉, NR₈COR₉, NR₈CO₂R₉, NR₈SO₂R₉, CONR₈R₉, SO₂R₈ ou SO₂NR₈R₉;
R₇ représente un groupe choisi parmi un thiènyle, isoxazolyle, pyrazolyle, phényle, pyridinyle, pyrimidinyle, naphtalènyle, quinolinyle, isoquinolinyle, le ou les groupes R₇ pouvant être substitués par un ou plusieurs groupes R₆ identiques ou différents l'un de l'autre ;
R₈ et R₉ représentent indépendamment l'un de l'autre un atome d'hydrogène ou un groupe C₁₋₆-alkyle, ou forment avec le ou les atomes qui les portent un cycle choisi parmi les cycles pyrrolidine, pipéridine, morpholine ; à l'état de base ou de sel d'addition à un acide.

6. Composé de formule (I) selon l'une quelconque des revendications 1 à 5 **caractérisé en ce que** R₃ représente un atome d'hydrogène, un groupe C₁₋₆-alkyle ou un groupe trifluorométhyle; à l'état de base ou de sel d'addition à un acide.

7. Composé de formule (I) selon l'une quelconque des revendications 1 à 6 **caractérisé en ce que**
R₄ représente un thiazole éventuellement substitué par un ou plusieurs substituants choisis parmi un atome d'halogène, un groupe C₁₋₆-alkyle, C₁₋₆-haloalkyle, pyridinyle, CONR₈R₉ ; R₈ et R₉ représentent indépendamment l'un de l'autre un atome d'hydrogène ou un groupe C₁₋₆-alkyle ;
à l'état de base ou de sel d'addition à un acide.

8. Procédé de préparation d'un composé de formule (I) selon l'une quelconque des revendications 1 à 7, comprenant l'étape consistant à
faire réagir une amine de formule générale (II), 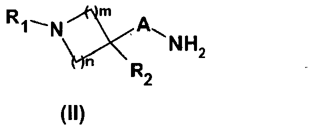 dans laquelle A, R₁, R₂, m et n sont tels que définis dans la formule générale (I) selon la revendication 1,
avec un carbonate de formule générale (III) 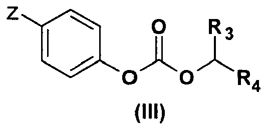 dans laquelle Z représente un atome d'hydrogène ou un groupe nitro, R₃ et R₄ sont tels que définis dans la formule générale (I) selon la revendication 1,
en présence d'une base, dans un solvant à une température comprise entre la température ambiante et la température de reflux du solvant.

9. Procédé de préparation d'un composé de formule (I) selon l'une quelconque des revendications 1 à 7, comprenant l'étape consistant à
faire réagir une amine de formule générale (II), 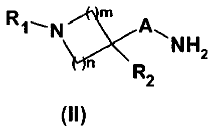 dans laquelle A, R₁, R₂, m et n sont tels que définis dans la formule générale (I) selon la revendication 1,
avec le chloroformiate de phényle ou de 4-nitro-phényle,
en présence d'une base, dans un solvant à une température comprise entre 0°C et la température ambiante,
pour conduire au dérivé carbamate de formule générale (IV), 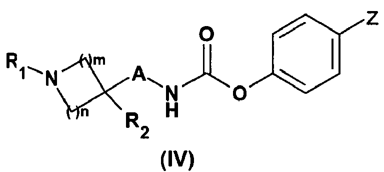 dans laquelle A, R₁, R₂, m et n sont tels que définis dans la formule générale (I) selon la revendication 1, et Z représente un atome d'hydrogène ou un groupe nitro ;
puis à transformer le dérivé carbamate de formule générale (IV) ainsi obtenu en composé de formule générale (I), par action d'un alcool de formule générale HOCHR₃R₄ (IIIa), dans laquelle R₃ et R₄ sont tels que définis dans la formule générale (I) selon la revendication 1,
en présence d'une base, dans un solvant à une température comprise entre la température ambiante et la température de reflux du solvant.

10. Composé de formule (I) selon l'une quelconque des revendications 1 à 7, à l'état de base ou de sel d'addition à un acide pharmaceutiquement acceptable, pour son utilisation comme médicament.

11. Composition pharmaceutique contenant au moins un composé de formule (I) selon l'une quelconque des revendications 1 à 7, à l'état de base ou de sel d'addition à un acide pharmaceutiquement acceptable et éventuellement un ou plusieurs excipients pharmaceutiquement acceptables.

12. Utilisation d'un composé de formule (I) selon l'une quelconque des revendications 1 à 7, à l'état de base ou de sel d'addition à un acide pharmaceutiquement acceptable, pour la préparation d'un médicament destiné à prévenir ou à traiter une pathologie dans laquelle les cannabinoïdes endogènes, sont impliqués.

13. Utilisation d'un composé de formule (I) selon l'une quelconque des revendications 1 à 7, à l'état de base ou de sel d'addition à un acide pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

## Claims

1. Compound of the formula (I) 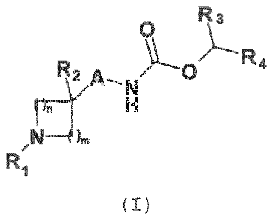 in which
R₂ represents a hydrogen or fluorine atom, or a hydroxyl, cyano, trifluoromethyl, C₁₋₆ alkyl, C₁₋₆ alkoxy or a NR₈R₉ group;
n represents an integer equal to 1, 2 or 3 and m represents an integer equal to 1 or 2;
A represents a covalent bond or a C₁₋₈ alkylene group;
R₁ represents a group R₅ which is optionally substituted by one or more groups R₆ and/or R₇;
R₅ represents a group selected from a phenyl, pyridinyle, pyridazinyl, pyrimidinyl, pyrazinyl, triazinyl, naphthalenyl, quinolinyl, isoquinolinyl, phthalazinyl, quinazolinyl, quinoxalinyl, cinnolinyl or naphthyridinyl;
R₆ represents a halogen atom, a cyano, -CH₂CN, nitro, hydroxyl, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene or C₃₋₇-cycloalkyl-C₁₋₃-alkylene-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ or -O-(C₁₋₃-alkylene)-O- group;
R₇ represents a group selected from a furanyl, pyrrolyl, thienyl, oxazolyl, isoxazolyl, thiazolyl, isothiazolyl, imidazolyl, pyrazolyl, oxadiazole, thiadiazole, phenyl, pyridinyl, pyridazinyl, pyrimidinyl, pyrazinyl, triazine, naphthalenyl, quinolinyl, isoquinolinyl, phthalazinyl, quinazolinyl, quinoxalinyl, cinnolinyl, naphthyridinyl, imidazopyrimidinyl, thienopyrimidinyl, benzofuranyl, benzothienyl, benzimidazolyl, benzoxazolyl, benzisoxazolyl, benzothiazolyl, benzisothiazolyl, indolyl, isoindolyl, indazolyl, pyrrolopyridinyl, furopyridinyl, thienopyridinyl, imidazopyridinyl, pyrazolopyridinyl, oxazolopyridinyl, isoxazolopyridinyl, thiazolopyridinyl, phenyloxy, benzyloxy or pyrimidinoxy, the group or groups R₇ being able to be substituted by one or more groups R₆ which are identical or different from one another;
R₃ represents a hydrogen or fluorine atom, a C₁₋₆ alkyl group or a trifluoromethyl group;
R₄ represents a thiazole which is optionally substituted by one or more substituents selected from a halogen atom, a C₁₋₆-alkyl, C₁₋₆-haloalkyl , C₃₋₇-cycloalkyl, C₃₋₇-cycloalkyl-C₁₋₃-alkylene, C₁₋₆-haloalkoxy, cyano, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉, -O-(C₁₋₃-alkylene)-O-, phenyl, phenyloxy, benzyloxy, pyridinyl, pyrazinyl, pyridazinyl, triazinyl or pyrimidinyl group, it being possible for the phenyl, phenyloxy, pyridinyl, pyrazinyl, pyridazinyl, triazinyl and pyrimidinyl groups to be substituted by one or more substituents selected from a halogen atom, a cyano, nitro, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cydoalkyl-C₁₋₃ alkylene group;
R₈ and R₉ represent independently of one another a hydrogen atom or a C₁₋₆-alkyl group,
or form, with the atom or atoms bearing them,
in the case of NR₈R₉, a ring selected from azetidine, pyrrolidine, piperidine, morpholine, thiomorpholine, azepine, oxazepine or piperazine rings, this ring being optionally substituted by a C₁₋₆ alkyl or benzyl group;
in the case of NR₈COR₉, a lactam ring; in the case of NR₈CO₂R₉, an oxazolidinone, oxazinone or oxazepinone ring; in the case of NR₈SO₂R₉, a sultam ring; in the case of NR₈SO₂NR₈R₉, a thiazolidine dioxide or thiadiazinane dioxide ring;
in the form of a base or an addition salt with an acid;
the following compounds being excluded:
- methyl 2-(3-{[2-(4-chlorophenyl)-4-methylthiazol-5-yl]methoxycarbony)amino}piperidin-1-yl)benzoate;
- 2-(3-{[2-(4-chlorophenyl)-4-methylthiazol-5-yl]methoxycarbonylamino}piperidin-1-yl)benzoic acid;
- methyl 3-(3-{[2-(4-chlorophenyl)-4-methylthiazol-5-yl]methoxycarbonylamino}piperidin-1-yl)benzoate;
- 3-(3-{[2-(4-chlorophenyl)-4-methylthiazol-5-yl]methoxycarbonylamino}piperidin-1-yl)benzoic acid.

2. Compound of formula (I) according to Claim 1, **characterized in that** R₂ represents a hydrogen atom;
in the form of a base or an addition salt with an acid.

3. Compound of formula (I) according to Claim 1 or 2, **characterized in that** n represents an integer equal to 1 or 2 and m represents an integer equal to 2;
in the form of a base or an addition salt with an acid.

4. Compound of formula (I) according to any one of Claims 1 to 3, **characterized in that** A represents a C₁₋₈ alkylene group; in the form or a base or an addition salt with an acid.

5. Compound of formula (I) according to any one of Claims 1 to 4, **characterized in that** R₁ represents a group R₅ which is optionally substituted by one or more groups R₆ and/or R₇;
R₅ represents a pyridinyl or quinolinyl group; group;
R₆ represents a halogen atom, a cyano, -CH₂CN, C₁₋₆-alkyl, C₁₋₆-alkoxy, C₁-₆-haloalkyl, C₃₋₇-cycloalkyl or C₃₋₇-cycloalkyl-C₁₋₃-alkylene-O-, NR₈R₉, NR₈COR₉, NR₈CO₂R₉, NR₈SO₂R₉, CONR₈R₉, SO₂R₈ or SO₂NR₈R₉ group; R₇ represents a group selected from a thienyl, isoxazolyl, pyrazolyl, phenyl, pyridinyl, pyrimidinyl, naphthalenyl, quinolinyl or isoquinolinyl, it being possible for the group or groups R₇ to be substituted by one or more groups R₆ which are identical or different from one another;
R₈ and R₉ represent independently of one another a hydrogen atom or a C₁₋₆-alkyl group, or form with the atom or atoms bearing them a ring selected from pyrrolidine, piperidine and morpholine rings; in the form of a base or an addition salt with an acid.

6. Compound of formula (I) according to any one of Claims 1 to 5, **characterized in that** R₃ represents a hydrogen atom, a C₁₋₆-alkyl group or a trifluoromethyl group; in the form of a base or an addition salt with an acid.

7. Compound of formula (I) according to any one of Claims 1 to 6, **characterized in that**
R₄ represents a thiazole which is optionally substituted by one or more substituents selected from a halogen atom, a C₁₋₆-alkyl, C₁₋₆-haloalkyl, pyridinyl and CONR₈R₉ group; R₈ and R₉ represent independently of one another a hydrogen atom or a C₁₋₆-alkyl group;
in the form of a base or an addition salt with an acid.

8. Process for preparing a compound of formula (I) according to any one of Claims 1 to 7, comprising the step of
reacting an amine of general formula (II) 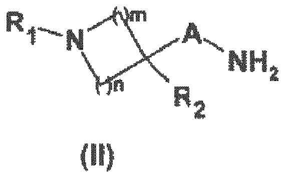 in which A, R₁, R₂, m and n are as defined in the general formula (I) according to Claim 1,
with a carbonate of general formula (III) 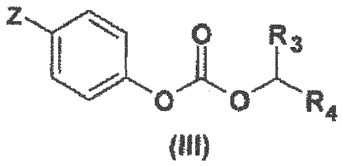 in which Z represents a hydrogen atom or a nitro group, R₃ and R4 are as defined in the general formula (I) according to Claim 1,
in the presence of a base, in a solvent at a temperature between the ambient temperature and the reflux temperature of the solvent.

9. Process for preparing a compound of formula (I) according to any one of Claims 1 to 7, comprising the step of
reacting an amine of general formula (II) 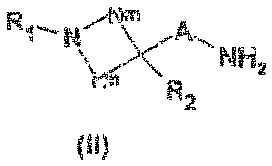 in which A, R₁, R₂, m and n are as defined in the general formula (I) according to Claim 1,
with phenyl or 4-nitrophenyl chloroformate,
in the presence of a base, in a solvent at a temperature between 0°C and the ambient temperature,
to give the carbamate derivative of general formula (IV) 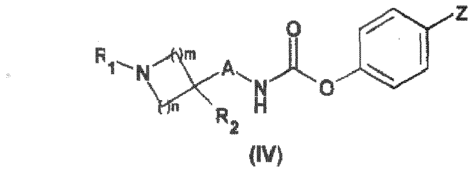 in which A, R₁, R₂, m and n are as defined in the general formula (I) according to Claim 1, and Z represents a hydrogen atom or a nitro group; then converting the carbamate derivative of general formula (IV) thus obtained into a compound of general formula (I), by the action of an alcohol of general formula HOCHR₃R₄ (IIIa), in which R₃ and R₄ are as defined in general formula (I) according to Claim 1,
in the presence of a base, in a solvent at a temperature between the ambient temperature and the reflux temperature of the solvent.

10. Compound of formula (I) according to any one of Claims 1 to 7, in the form of a base or an addition salt with a pharmaceutically acceptable acid, for use thereof as a medicinal product.

11. Pharmaceutical composition comprising at least one compound of formula (I) according to any one of Claims 1 to 7, in the form of a base or an addition salt with a pharmaceutically acceptable acid, and, optionally, one or more pharmaceutically acceptable excipients.

12. Use of a compound of formula (I) according to any one of Claims 1 to 7, in the form of a base or an addition salt with a pharmaceutically acceptable acid, for preparing a medicinal product intended for preventing or treating a pathology involving endogenous cannabinoids.

13. Use of a compound of formula (I) according to any one of Claims 1to 7, in the form of a base or an addition salt with a pharmaceutically acceptable acid, for preparing a medicinal product intended for preventing or treating acute or chronic pain, dizziness, vomiting, nausea, eating disorders, neurological and psychiatric pathologies, acute or chronic neurodegenerative diseases, epilepsy, sleep disorders, cardiovascular diseases, renal ischaemia, cancers, disorders of the immune system, allergic diseases, infectious parasitic, viral or bacterial diseases, inflammatory diseases, osteoporosis, ocular conditions, pulmonary conditions, gastrointestinal diseases or urinary incontinence.

## Patentansprüche

1. Verbindung nach der Formel (I) 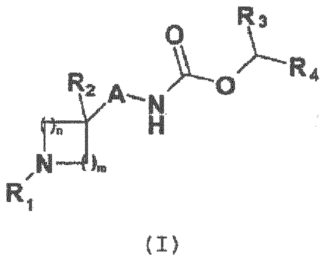 wobei
R₂ für ein Wasserstoff-, Fluoratom oder eine Hydroxyl-, Cyano-, Trifluormethyl, C₁₋₆-Alkyl-, C₁₋₆-Alkoxy-, NR₈R₉-Gruppe steht;
n für eine ganze Zahl steht, die gleich 1, 2 oder 3 ist, und m für eine ganze Zahl steht, die gleich 1 oder 2 ist;
A für eine kovalente Bindung oder eine C₁₋₈-Alkylengruppe steht;
R₁ für eine R₅-Gruppe steht, die möglicherweise mit einer oder mehreren R₆ - und/oder R₇-Gruppen substituiert ist ;
R₅ für eine Gruppe steht, die aus einem Phenyl, Pyridinyl, Pyridazinyl, Pyrimidinyl, Pyrazinyl, Triazinyl, Naphthalenyl, Chinolinyl, Isochinolinyl, Phthalazinyl, Chinazolinyl, Chinoxalinyl, Cinnolinyl, Naphthyridinyl ausgewählt ist;
R₆ für ein Halogenatom, eine Cyano-, -CH₂CN-, Nitro-, Hydroxyl-, C₁₋₆-Alkyl-, C₁₋₆-Alkoxy-, C₁₋₆-Thioalkyl-, C₁₋₆-Haloalkyl-, C₁₋₆-Haloalkoxy-, C₁₋₆-Halothioalkyl-, C₃₋₇-Cycloalkyl-, C₃₋₇-Cycloalkyl-C₁₋₃-alkylen-, C₃-₇-Cycloalkyl-C₁₋₃-alkylen-O-, NR₈R₉-, NR₈COR₉-, NR₈CO₂R₉-, NR₈SO₂R₉-, NR₈SO₂NR₈R₉-, COR₈-, CO₂R₈-, CONR₈R₉-, SO₂R₈-, SO₂NR₈R₉- oder -O-(C₁₋₃-Alkylen)-O-Gruppe steht ;
R₇ für eine Gruppe steht, die aus einem Furanyl, Pyrrolyl, Thienyl, Oxazolyl, Isoxazolyl, Thiazolyl, Isothiazolyl, Imidazolyl, Pyrazolyl, Oxadiazol, Thiadiazol, Phenyl, Pyridinyl, Pyridazinyl, Pyrimidinyl, Pyrazinyl, Triazin, Naphtalenyl, Chinolinyl, Isochinolinyl, Phthalazinyl, Chinazolinyl, Chinoxalinyl, Cinnolinyl, Naphthyridinyl, Imidazopyrimidinyl, Thienopyrimidinyl, Benzofuranyl, Benzothienyl, Benzimidazolyl, Benzoxazolyl, Benzisoxazolyl, Benzothiazolyl, Benzisothiazolyl, Indolyl, Isoindolyl, Indazolyl, Pyrrolopyridinyl, Furopyridinyl, Thienopyridinyl, Imidazopyridinyl, Pyrazolopyridinyl, Oxazolopyridinyl, Isoxazolopyridinyl, Thiazolopyridinyl, Phenyloxy, Benzyloxy, Pyrimidinoxy ausgewählt ist; wobei die R₇-Gruppe (n) mit einer oder mehreren R₆-Gruppen substituiert sein kann/können, die gleichartig oder verschiedenartig sind;
R₃ für ein Wasserstoff-, Fluoratom, eine C₁₋₆-Alkyl- oder eine Trifluormethylgruppe steht;
R₄ für ein Thiazol steht, das möglicherweise mit einem oder mehreren Substituenten substituiert ist, welche aus einem Halogenatom, einer C₁₋₆-Alkyl-, C₁₋₆-Haloalkyl-, C₃₋₇-Cycloalkyl-, C₃₋₇-Cycloalkyl-C₁₋₃-alkylen-, C₁₋₆-Haloalkoxy-, Cyano-, NR₈R₉-, NR₈COR₉ -, NR₈CO₂R₉-, NR₈SO₂R₉-, NR₈SO₂NR₉R₉-, COR₈-, CO₂R₈-, CONR₈R₉-, SO₂R₈-, SO₂NR₈R₉-, -O-(C₁₋₃-Alkylen)-O-, Phenyl-, Phenyloxy-, Benzyloxy-, Pyridinyl-, Pyrazinyl-, Pyridazinyl-, Triazinyl- oder Pyrimidinylgruppe ausgewählt ist; wobei die Phenyl-, Phenyloxy-, Pyridinyl-, Pyrazinyl-, Pyridazinyl-, Triazinyl- und Pyrimidinylgruppen mit einem oder mehreren Substituenten substituiert sein können, die aus einem Halogenatom, einer Cyano-, Nitro-, C₁₋₆-Alkyl-, C₁₋₆-Alkoxy-, C₁₋₆-Thioalkyl-, C₁₋₆-Haloalkyl-, C₁₋₆-Haloalkoxy-, C₁₋₆-Halothioalkyl-, C₃₋₇-Cycloalkyl-, C₃₋₇-Cycloalkyl-C₁₋₃-alkylengruppe ausgewählt sind ;
R₈ und R₉, unabhängig voneinander, für ein Wasserstoffatom oder eine C₁₋₆-Alkylgruppe stehen;
oder mit dem/den Atom(en), an welche (s) sie gebunden sind, Folgendes bilden:
im Falle von NR₈R₉ einen Ring, der aus den Ringen, Pyrrolidin, Piperidin, Morpholin, Thiomorpholin Azepin, Oxazepin oder Piperazin ausgewählt ist, wobei diese Ring möglicherweise mit einer C₁₋₆-Alkyl- oder Benzylgruppe substituiert ist ;
im Falle von NR₈COR₉ einen Lactam-Ring; im Falle von NR₈CO₂R₉ einen Oxazolidinon-, Oxazinon- oder Oxazepinon-Ring;
im Falle von NR₈SO₂R₉ einen Sultam-Ring ; im Falle von NR₈SO₂NR₈R₉ einen Thiazolidindioxid-oder Thiadiazinandioxid-Ring;
in Form einer Base oder eines Säureadditionssalzes;
wobei die folgenden Verbindungen ausgeschlossen sind:
- Methyl-2-(3-{[2-(4-chlorphenyl)-4-methylthiazol-5-yl]methoxycarbonylamino)piperidin-1 yl)benzoat;
- 2-(3-{[2-(4-Chlorphenyl)-4-methylthiazol-5-yl]methoxycarbonylamino}piperidin-1-yl)benzoesäure;
- Methyl-3-(3-{[2-(4-chlorphenyl)-4-methylthiazol-5-yl]methoxycarbonylamino}piperidin-1-yl)benzoat;
- 3-(3-{[2-(4-Chlorphenyl)-4-methylthiazol-5-yl]methoxycarbonylamino}piperidin-1-yl)benzoesäure.

2. Verbindung nach Formel (I) nach Anspruch 1, **dadurch gekennzeichnet, dass** R₂ für ein Wasserstoffatom steht; in Form einer Base oder eines Säureadditionssalzes.

3. Verbindung nach Formel (I) nach Anspruch 1 oder 2, **dadurch gekennzeichnet, dass** n für eine ganze Zahl steht, die gleich 1 oder 2 ist, und m für eine ganze Zahl steht, die gleich 2 ist;
in Form einer Base oder eines Säureadditionssalzes.

4. Verbindung nach Formel (I) nach einem beliebigen der Ansprüche 1 bis 3, **dadurch gekennzeichnet, dass** A für eine C₁₋₈-Alkylengruppe steht; in Form einer Base oder eines Säureadditionssalzes.

5. Verbindung nach Formel (I) nach einem beliebigen der Ansprüche 1 bis 4, **dadurch gekennzeichnet, dass** R₁ für eine R₅-Gruppe steht, die möglicherweise mit einer oder mehreren R₆- und/oder R₇-Gruppen substituiert ist;
R₅ für eine Pyridinyl- oder Chinolinylgruppe steht;
R₆ für ein Halogenatom, eine Cyano-, -CH₂CN-, C₁₋₆-Alkyl-, C₁₋₆-Alkoxy-, C₁₋₆-Haloalkyl-, C₃₋₇-Cycloalkyl-, C₃₋₇-Cycloalkyl-C₁₋₃-alkylen-O-, NR₈R₉-, NR₈COR₉-, NR₈CO₂R₉-, NR₈SO₂R₉-, CONR₈R₉-, SO₂R₈- oder SO₂NR₈R₉-Gruppe steht;
R₇ steht für eine Gruppe, die aus Thienyl, Isoxazolyl, Pyrazolyl, Phenyl, Pyridinyl, Pyrimidinyl, Naphthalenyl, Chinolinyl, Isochinolinyl ausgewählt ist, wobei die R₇-Gruppe(n) mit einer oder mehreren R₆-Gruppen substituiert sein kann/können, welche gleichartig oder verschiedenartig sein können;
R₈ und R₉ unabhängig voneinander für ein Wasserstoffatom oder eine C₁₋₆-Alkylgruppe stehen, oder mit dem oder den Atom(en), an welche (s) sie gebunden sind, einen Ring bilden, der aus den Ringen Pyrrolidin, Piperidin, Morpholin ausgewählt ist ; in Form einer Base oder eines Säureadditionssalzes.

6. Verbindung nach Formel (I) nach einem beliebigen der Ansprüche 1 bis 5, **dadurch gekennzeichnet, dass** R₃ für ein Wasserstoffatom, eine C₁₋₆-Alkylgruppe oder eine Trifluormethylgruppe steht; in Form einer Base oder eines Säureadditionssalzes.

7. verbidung nach Formel (I) nach einem beliebigen der Ansprüche 1 bis 6, **dadurch gekennzeichnet, dass**
R₄ für ein Thiazol steht, das möglicherweise mit einem oder mehreren Substituenten substituiert ist, welche aus einem Halogenatom, einer C₁₋₆-Alkyl-, C₁₋₆-Haloalkyl-, Pyridinyl-, CONR₈R₉-Gruppe ausgewählt ist; wobei R₈ und R₉ unabhängig voneinander für ein Wasserstoffatom oder eine C₁₋₆-Alkylgruppe stechen;
in Form einer Base oder eines Säureadditionssalzes.

8. Herstellungsverfahren für eine Verbindung nach Formel (I) nach einem beliebigen der Ansprüche 1 bis 7, welches den Schritt umfasst, der darin besteht,
ein Amin nach der allgemeinen Formel (II), 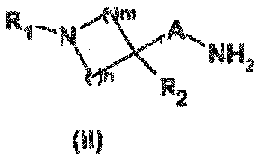 in welcher A, R₁, R₂, m und n den Begriffsbestimmungen für die allgemeine Formel (I) nach Anspruch 1 entsprechen,
mit einem Carbonat umzusetzen, welches der allgemeinen Formel (III) 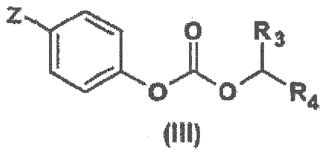 entspricht, wobei Z für ein Wasserstoffatom oder eine Nitrogruppe steht, R₃ und R₄ der Begriffsbestimmung für die allgemeine Formel (I) nach Anspruch 1 entsprechen,
und zwar in Gegenwart einer Base, in einem Lösemittel mit einer Temperatur im Bereich von der Raumtemperatur bis zur Rückflusstemperatur des Lösemittels.

9. Herstellungsverfahren für eine Verbindung nach Formel (I) nach einem beliebigen der Ansprüche 1 bis 7, welches den Schritt umfasst, der darin besteht,
ein Amin nach der allgemeinen Formel (II), 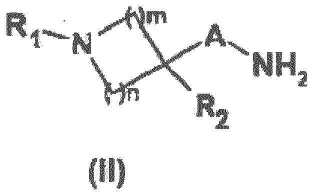 in welcher A, R₁, R₂, m und n den Begriffsbestimmungen für die allgemeine Formel (I) nach Anspruch 1 entsprechen,
mit Phenyl- oder 4-Nitrophenylchlorformiat umzusetzen,
und zwar in Gegenwart einer Base, in einem Lösemittel mit einer Temperatur im Bereich von 0 °C bis zur Raumtemperatur,
um ein Carbamatderivat zu erhalten, das der allgemeinen Formel (IV) 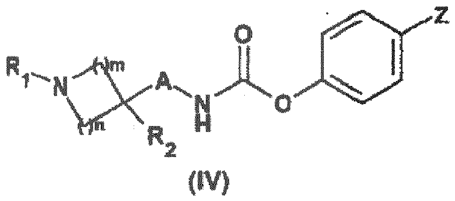 entspricht, wobei A, R₁, R₂, m und n den Begriffsbestimmungen für die allgemeine Formel (I) nach Anspruch 1 entsprechen, und Z für ein Wasserstoffatom oder eine Nitrogruppe steht;
und das auf diese Weise erhalten Carbamatderivat der allgemeinen Formel (IV) anschließend in eine Verbindung der allgemeinen Formel (I) umzuwandeln, durch Einwirkung, eines Alkohols der allgemeinen Formel HOCHR₃R₄ (IIIa), wobei R₃ und R₄ den Begriffsbestimmungen für die allgemeine Formel (I) nach Anspruch 1 entsprechen,
und zwar in Gegenwart einer Base, in einem Lösemittel mit einer Temperatur im Bereich von der Raumtemperatur bis zur Rückflusstemperatur des Lösemittels.

10. Verbindung nach Formel (I) nach einem beliebigen der Ansprüche 1 bis 7 in Form einer Base oder eines pharmazeutisch unbedenklichen Säureadditionssalzes zur Verwendung derselben als Arzneimittel.

11. Pharmazeutische Zusammensetzung, welche mindestens eine Verbindung nach Formel (I) nach einem beliebigen der Ansprüche 1 bis 7 in Form einer Base oder eines pharmazeutisch unbedenklichen Säureadditionssalzes sowie möglicherweise einen oder mehrere pharmazeutisch unbedenkliche Trägerstoff (e) enthält.

12. Verwendung einer Verbindung nach Formel (I) nach einem beliebigen der Ansprüche 1 bis 7 in Form einer Base oder eines pharmazeutisch unbedenklichen Säureadditionssalzes zur Herstellung eines Arzneimittels, das dazu bestimmt ist, ein Krankheitsbild zu behandeln oder diesem vorzubeugen, bei welchem endogene Cannabinoide eine Rolle spielen.

13. Verwendung einer Verbindung nach Formel (I) nach einem beliebigen der Ansprüche 1 bis 7 in Form einer Base oder eines pharmazeutisch unbedenklichen Säureadditionssalzes zur Herstellung eines Arzneimittels, das dazu bestimmt ist, akute oder chronische Schmerzen, Schwindelgefühl, Erbrechen, Übelkeit, Essstörungen, neurologische oder sychiatrische Erkrankungen, akute oder chronische neurodegenerative Krankheiten, Epilepsie, Schlafstörungen, Herz-Kreislauf-Krankheiten, renale Ischämie, Krebserkrankungen, Störungen des Immunsystems, allergische Krankheiten, Infektionskrankheiten, die durch Parasiten, Viren oder Bakterien ausgelöst werden, entzündliche Erkrankungen, Osteoporose, Augenerkrankungen, Lungenerkrankungen, Krankheiten des Magen-DarmTraktes oder Harninkontinenz zu behandeln oder diesen vorzubeugen.
